# EUROPÄISCHE PATENTSCHRIFT

(11) **EP 4 304 861 B1**
(45) Veröffentlichungstag und Bekanntmachung des Hinweises auf die Patenterteilung: **19.02.2025**
(21) Anmeldenummer: 22710402.3
(22) Anmeldetag: 07.03.2022
(51) Int. Cl.: B32B 27/08, B32B 3/08, B32B 7/02, B32B 27/18, B32B 27/30, B32B 27/32, C08L 23/08, C08L 23/12, C08L 23/14, C08L 25/08

(54) **FLEXIBLE HITZESTERILISIERBARE NON-PVC MEHRSCHICHTFOLIE FÜR MEDIZINISCHE VERPACKUNGEN**
FLEXIBLE HEAT-STERILIZABLE NON-PVC MULTILAYER FILM FOR MEDICAL PACKAGING
FEUILLE MULTICOUCHE NON PVC FLEXIBLE POUVANT ÊTRE STÉRILISÉE À LA CHALEUR POUR EMBALLAGES MÉDICAUX

(30) Priorität: 09.03.2021 EP 21161586
(43) Veröffentlichungstag der Anmeldung: 17.01.2024
(73) Patentinhaber: PolyCine GmbH, 66578 Schiffweiler (DE)
(72) Erfinder: HOLZER, Susanne, 66564 Ottweiler (DE); GROSS, René, 66538 Neunkirchen (DE)
(74) Vertreter: Rippel, Hans Christoph
(86) Internationale Anmeldenummer: PCT/EP2022/055706
(87) Internationale Veröffentlichungsnummer: WO 2022/189335

(56) Entgegenhaltungen:
- WO-A1-2020/127227
- DE-A1- 10 361 851
- DE-U1- 20 320 212

## Beschreibung

Die Erfindung betrifft hitzesterilisierbare Mehrschichtfolien, enthaltend aliphatische Polyolefine, ein Verfahren zu deren Herstellung und deren Verwendung zur Herstellung von medizinischen Verpackungen, sowie medizinische Verpackungen, enthaltend solche Mehrschichtfolien. Die Mehrschichtfolien sind heißdampfsterilisierbar und zeichnen sich bei Verwendung für medizinische Verpackungen durch eine geringe Adsorptionsneigung von Medikamenten oder medizinischen Lösungen, eine temperaturresistente Außenschicht und eine ausreichende Haftung zwischen den einzelnen Schichten aus.

Mehrschichtfolien finden seit vielen Jahren ein breites Anwendungsgebiet, z.B. in der Lebensmittel-Industrie aber auch im medizinisch/pharmazeutischen Bereich beispielsweise als Sekundär-Packmittel (Umverpackung) oder Primär-Packmittel für Lösungsbeutel, Trockenkonzentrate und Medikamente in Tablettenform.

Einige Mehrschichtfolien sind zu flexiblen Verpackungen verarbeitbar, welche beispielsweise als Beutel zum Verpacken und zum Verabreichen von medizinischen Lösungen geeignet sind. Als gängige Praxis sind derzeit medizinische Lösungen z. B. Infusionslösungen für die parenterale Verabreichung, in flexiblen Einwegbeuteln aus Polyvinylchlorid- (PVC) oder non-PVC-Materialien am Markt.

Elastische PVC-Materialien sind gesundheitsbedenklich, da sie Weichmacher enthalten, die wieder freigesetzt werden können. Daher ist man bestrebt, PVC durch non-PVC-Materialien zu ersetzen.

Flexible medizinische Beutel müssen neben der Fähigkeit zum Kollabieren, das ein vollständiges Auslaufen des Beutels gewährleistet, weitere Leistungskriterien wie Transparenz, Heißsterilisationsfähigkeit bei 121°C, ausreichende mechanische Festigkeit vor allem bei dynamischer Belastung im Schweißnahtbereich, gute Wasserdampfbarriere, Belastungsfähigkeit für übliche Druckmanschettenanwendungen z. B. Druckinfusionen und aus pharmazeutischer Sicht eine möglichst geringe Beeinflussung des Beutelinhalts durch die Verpackung aufweisen.

Mehrschichtfolien mit einem Schichtaufbau auf Basis von Polyolefinen haben sich bezüglich dieser Erfordernisse als vorteilhaft erwiesen.

DE-A 10361851 und WO 2020/127227 A1 beschreiben eine hitzesterilisierbare 3-lagige Mehrschichtfolie zur Herstellung von medizinischen Beuteln, deren Außenschicht aus mit Impact Modifiern modifiziertem Polypropylen-Homopolymer, deren Mittelschicht aus mit Impact Modifiern modifiziertem Polypropylen-Terpolymer, und deren Innenschicht aus mit Impact Modifiern modifiziertem Polypropylen-Terpolymer und/oder Polypropylen-Copolymer, besteht. Geeignete Impact-Modifier sind Styrol-Blockcopolymere (z.B. SEB) und Ethylen/α-Olefin-Copolymere. Beispielhafte Folien weisen eine Mittelschicht aus 75 Gew.-% PP-Terpolymer, 20 Gew.-% SEBS-Blockcopolymer und 5 Gew.-% PE-Plastomer (Ethylen/Octen-Copolymer), und eine Innenschicht aus 85 bzw. 75 Gew.-% PP-Terpolymer, 15 bzw. 20 Gew.-% SEB-Blockcopolymer und 0 bzw. 5 Gew.-% PE-Plastomer auf.

DE 203 20 212 A1 beschreibt eine hitzesterilisierbare 3-lagige Mehrschichtfolie hergestellt durch Coextrusion zur Verwendung für medizinische Beutel. Beispielhafte Folien weisen eine Außenschicht aus 97 Gew.-% Polypropylen-Homopolymer und 3 Gew.-% SEBS-Blockcopolymer, eine Mittelschicht aus 80 Gew.-% EXCELLEN - einem heterophasigen Copolymer auf Basis von Polypropylen und Polyethylen, und 20 Gew.-% SEBS-Blockcopolymer, und eine Innenschicht aus 75 Gew.-% PP-Terpolymer, 20 Gew.-% SEBS-Blockcopolymer und 5 Gew.-% PE-Plastomer auf.

KR-A 2010-0101332 offenbart Mehrschichtfolien mit exzellenter Haftung zwischen den Schichten, hergestellt durch Coextrusion einer Polypropylen-Schicht und einer Polyethylen-Schicht, wobei die Polypropylen-Schicht ein Blend mit Polyethylen (Anteil 40 bis 50 Gew.-%) oder die Polyethylen-Schicht ein Blend mit Polypropylen (Anteil 20 bis 50 Gew.-%) ist.

EP-A 2231775 befasst sich mit einer Mehrschichtfolie zur Verwendung als Behälter für medizinische Lösungen, umfassend bevorzugt eine äußere Schicht aus Propylenhomopolymer; eine Mittelschicht aus 30 bis 70 Gew.-% Propylen-basiertes Polymer (z.B. Propylen-Ethylen-Buten-Terpolymer) und 30 bis 70 Gew.-% thermoplastisches Elastomer (z.B. SEBS); und eine innere Schicht aus 50 bis 70 Gew.-% Propylen-Copolymer, 5 bis 20 Gew.-% Polyethylen (z.B. HDPE), und 10 bis 45 Gew.-% thermoplastisches Elastomer (z.B. SEBS).

EP-A 0229475 beschreibt eine Mehrschichtfolie für medizinische Behälter, die bevorzugt 3-lagig ist, enthaltend (a) eine erste (= innenliegende) heißsiegelfähige Schicht aus einem Blend vorzugsweise aus 40 bis 70 Gew.-% Propylen-Copolymer, 10 bis 40 Gew.-% Ethylen-Propylen- oder Ethylen-1-Buten-Copolymer (Anteil 1-Buten vorzugsweise 5 bis 15 Gew.-%), und 5 bis 35 Gew.-% Elastomer (z.B. Ethylen-Copolymer, Styrol-Blockcopolymer); (b) eine zweite (mittlere) Schicht aus einem Blend von (i) Polyethylen (HDPE) (50 bis 90 Gew.-%) und (ii) einem Modifikator; und (c) eine dritte (äußere) Schicht aus einem Blend von (i) Polypropylen und (ii) einem Modifikator.

JP-A 2007/245490 beschreibt eine hitzesterilisierbare Mehrschichtfolie zur Verwendung für medizinische Beutel, welche eine innere Schicht hoher Dichte (0,89 bis 0,93 g/cm³) aus einem Blend eines - mittels eines Metallocen-Katalysators hergestellten - Ethylen/α-Olefin-Copolymers und HDPE (Anteil 10 bis 40 Gew.-%), und eine äußere Schicht aus einem Propylenpolymer enthält.

US 2012/0034404 A1 beschreibt eine Mehrschichtfolie für medizinische Verpackungen (z.B. Beutel) umfassend: eine äußere Schicht umfassend bevorzugt ein Propylen-Homopolymer; eine mittlere Schicht umfassend 10 bis 60 Gew.-% eines Propylen-Copolymers und 40 bis 90 Gew.-% eines thermoplastischen Elastomers; und eine innere Schicht aus: 60 bis 80 Gew.-% eines Propylen-Copolymers, 10 bis 30 Gew.-% Polyethylen und 1 bis 10 Gew.-% eines thermoplastischen Elastomers. Das Polyethylen ist bevorzugt ein Copolymer von Ethylen und einem α-Olefin mit einer Schmelztemperatur von 50 bis 120°C (Beispiele: 60°C); bevorzugt ist das Propylen-Copolymer ein Propylen-Ethylen-Buten-Terpolymer. Als thermoplastisches Elastomer wird bevorzugt ein hydriertes Styrol-Blockcopolymer eingesetzt.

Die Mehrschichtfolien gemäß dem Stand der Technik sind insbesondere in Bezug auf die Haftung zwischen den einzelnen Schichten und die Anforderungen an die Heißdampfsterilisierbarkeit bei 121°C noch verbesserungsbedürftig.

Aufgabe der vorliegenden Erfindung ist es daher, eine flexible, hitzesterilisierbare Mehrschichtfolie für medizinische Zwecke bereitzustellen, die eine gute Haftung zwischen den einzelnen Schichten und eine gute Schlagzähigkeit besitzt, und ferner eine geringe Adsorptionsneigung von Medikamenten oder medizinischen Lösungen an der dem Medikament oder der medizinischen Lösung zugewandten Oberfläche (Innenschicht) aufweist, und durch einfache Verfahren zu medizinischen Behältern (z.B. Beuteln) verarbeitet werden kann.

Ein Gegenstand der Erfindung ist eine hitzesterilisierbare Mehrschichtfolie, umfassend (bestehend aus)
a) eine erste Polymerschicht (A) enthaltend (bestehend aus) mindestens ein, vorzugsweise ein, mit mindestens einem, vorzugsweise einem, Impact Modifier modifiziertes Propylen-Homopolymer;
b) eine zweite Polymerschicht (B) enthaltend (bestehend aus):
   B1) 60 bis 85 Gew.-% - bezogen auf (B) - einer homogenen Zusammensetzung (= "Composite") (B1) bestehend aus:
      B11) 65 bis 85 Gew.-% - bezogen auf (B1) - Ethylen-Homopolymer, und
      B12) 15 bis 35 Gew.-%, bevorzugt 20 bis 30 Gew.-%, - bezogen auf (B1) - mindestens eines Ethylen-Copolymers, welches als Comonomer mindestens ein, vorzugsweise ein, alpha-Olefin mit 4 bis 12, bevorzugt 4 bis 8, besonders bevorzugt 4 bis 6 Kohlenstoffatomen, bevorzugt ausgewählt aus 1-Buten, 1-Penten, 1-Hexen und 4-Methyl-1-penten, vorzugsweise 1-Buten, enthält,
      und wobei Zusammensetzung (B1) eine Schmelztemperatur von > 125°C und eine Dichte von 945 bis 960 kg/m³ aufweist;
   B2) 11 bis 30 Gew.-% - bezogen auf (B) - mindestens eines Propylen-Terpolymers;
   B3) 4 bis 15 Gew.-% - bezogen auf (B) - mindestens eines Polyethylen-Elastomers, welches ein Copolymer von Ethylen mit mindestens einem, vorzugsweise einem, alpha-Olefin mit 4 bis 12, bevorzugt 7 bis 12, Kohlenstoffatomen ist;
c) eine mittlere Polymerschicht (C), die sich zwischen der ersten Polymerschicht (A) und der zweiten Polymerschicht (B) befindet, enthaltend (bestehend aus):
   C1) 61 bis 80 Gew.-%, bevorzugt 65 bis 80 Gew.-%, - bezogen auf (C) - mindestens eines Propylen-Terpolymers;
   C2) 15 bis 25 Gew.-% - bezogen auf (C) - mindestens eines Styrol-Blockcopolymer-Elastomers;
   C3) 4 bis 14 Gew.-%, bevorzugt 4 bis 10 Gew.-%, - bezogen auf (C) - mindestens eines Polyethylen-Elastomers, welches ein Copolymer von Ethylen mit mindestens einem, vorzugsweise einem, alpha-Olefin enthaltend 4 bis 12 Kohlenstoffatome ist.

Die in Gewichtsprozent (Gew.-%) angegebenen Anteile ergeben jeweils zusammen 100 Gew.-%.

Im Sinne der vorliegenden Erfindung sind unter den Struktureinheiten eines Monomers in einem (Co)Polymer die von dem einpolymerisierten Monomer abgeleiteten Struktureinheiten zu verstehen.

Der Begriff "hitzesterilisierbar" bedeutet, dass entsprechende Materialien einer Sterilisation bei erhöhten Temperaturen, vorzugsweise einer Dampfsterilisation unterzogen werden können. Mit der Sterilisation bezeichnet man Verfahren, durch die Materialien und Gegenstände von lebenden Mikroorganismen befreit werden. Den damit erreichten Zustand der Materialien und Gegenstände bezeichnet man als "steril". Bei der Dampfsterilisation der befüllten oder unbefüllten medizinischen Verpackungen verwendet man heißen Wasserdampf zur Sterilisation, welche typischerweise in einem Autoklaven durchgeführt wird. Dabei werden die medizinischen Verpackungen vorzugsweise 20 Minuten auf 121 °C bei 2 bar Druck im Wasserdampf erhitzt. Die Luft im Inneren des Autoklaven wird dabei vollständig durch Wasserdampf ersetzt.

Der Begriff "Mehrschichtfolie" bezieht sich auf thermoplastische Materialien in mehreren coextrudierten Polymerschichten, die miteinander zu einer Folie in Form einer laufenden Bahn oder eines Schlauchs verbunden sind.

Der Begriff "Impact Modifier" bezeichnet polymere Materialien, wie z.B. Styrol-Blockcopolymer Elastomere, Polyethylen-Elastomere und Polypropylen-Elastomere, die durch Einmischung im Schmelzezustand die Schlagzähigkeit des den Impact Modifier umgebenden Polymers verbessern.

Der Begriff "Schlagzähigkeit" bezeichnet die Eigenschaft eines Werkstoffes einer dynamischen Belastung zu wiederstehen. Nach der Norm DIN EN ISO 180:2013-08 kann die Izod-Schlagzähigkeit von Kunststoffen unter festgelegten Bedingungen gemessen werden.

Unter einer "homogenen Zusammensetzung" ist ein Composite von auf molekularer Ebene vermischten Stoffen (Komponenten) zu verstehen, die gemeinsam eine einzige Phase bilden.

Die "Schmelztemperatur" (Erweichungstemperatur, Tₘ) wird üblicherweise mittels DSC (Differential Scanning Calorimetry) bestimmt.

Die "Dichte" kann gemäß DIN EN ISO 1183-1 (2019-09) - Verfahren B) mit dem Flüssigkeitspyknometer bestimmt werden.

### Erste Polymerschicht (A)

Die erste Polymerschicht (A) der erfindungsgemäßen Mehrschichtfolie stellt definitionsgemäß die Polymerschicht dar, welche bei Verarbeitung der Folie zu einer Verpackung, welche vorzugsweise ein Beutel ist, auf der Außenseite der Verpackung befindlich ist. Demnach ist sie bei der Weiterverarbeitung der Folie zu Verpackungen im direkten Kontakt mit der Oberfläche des Schweißwerkzeuges und benötigt deshalb vorzugsweise eine hohe Schmelz- bzw. Erweichungstemperatur, die bevorzugt oberhalb von 125°C, besonders bevorzugt zwischen 127°C und 150°C, ganz besonders bevorzugt zwischen 130°C und 145°C liegt.

Die erste Polymerschicht (A) enthält mindestens ein, vorzugsweise ein, Polypropylen-Homopolymer, welches mit mindestens einem, vorzugsweise einem, Impact Modifier modifiziert ist.

Bevorzugt besteht die erste Polymerschicht (A) aus mindestens einem, vorzugsweise einem, Polypropylen-Homopolymer, welches mit mindestens einem, vorzugsweise einem, Impact Modifier modifiziert ist.

Weiterhin bevorzugt enthält die erste Polymerschicht (A) ein Polypropylen-Homopolymer, welches mit einem Impact Modifier modifiziert ist.

Die erste Polymerschicht (A) enthält (oder besteht aus) mindestens ein, vorzugsweise ein, Propylen-Homopolymer (Polypropylen), welches zur Verbesserung der (Kälte)Schlagzähigkeit generell mit 1 bis 30 Gew.-%, bevorzugt mit 2 bis 20 Gew.-%, besonders bevorzugt 2 bis 10 Gew.-%, insbesondere 3 bis 5 Gew.-%, mindestens eines Impact Modifiers modifiziert ist.

Die Herstellung von Propylen-Homopolymeren ist bekannt. Ferner sind Propylen-Homopolymere kommerziell erhältlich z.B. von Lyondell Basell Corporation, USA.

Bevorzugt enthält (oder besteht aus) die erste Polymerschicht (A) ein Propylen-Homopolymer, welches mit mindestens einem Impact Modifier, ausgewählt aus der Gruppe der Styrol-Blockcopolymere wie z. B. Styrol-Ethylen-Butylen-Styrol-Blockcopolymer (SEBS), Styrol-Ethylen-Propylen-Styrol-Blockcopolymer (SEPS), Styrol-Ethylen-Ethylen-Propylen-Styrol-Blockcopolymer (SEEPS), Styrol-Isopren-Styrol-Blockcopolymer (SIS) und Styrol-Butadien-Styrol-Blockcopolymer (SBS), bevorzugt SEBS und SEPS, insbesondere SEBS, und/oder aus der Gruppe der Copolymere von Ethylen mit mindestens einem alpha-Olefin mit 4 bis 12, bevorzugt 4 bis 8 Kohlenstoffatomen wie z.B. Ethylen-Butylen-Copolymere und/oder Ethylen-1-Octen-Copolymere, modifiziert ist.

Besonders bevorzugt enthält (oder besteht aus) die erste Polymerschicht (A) 90 bis 98 Gew.-%, insbesondere 95 bis 97 Gew.-%, eines Propylen-Homopolymers und 2 bis 10 Gew.-%, insbesondere 3 bis 5 Gew.-%, eines Styrol-Blockcopolymers und/oder eines Copolymers von Ethylen mit mindestens einem alpha-Olefin mit 4 bis 12, bevorzugt 4 bis 8 Kohlenstoffatomen.

In einer bevorzugten Ausführungsform enthält (oder besteht aus) die erste Polymerschicht (A) 95 bis 97 Gew.-% eines Polypropylen-Homopolymers und 3 bis 5 Gew.-% eines Styrol-Ethylen/Butylen-Blockcopolymers.

Die Gewichtsangaben für die Komponenten der ersten Polymerschicht (A) beziehen sich auf das Gesamtgewicht der ersten Polymerschicht (A).

### Zweite Polymerschicht (B)

Die zweite Polymerschicht (B) stellt definitionsgemäß die Polymerschicht dar, welche bei Verarbeitung der erfindungsgemäßen Mehrschichtfolie zu einer Verpackung, welche vorzugsweise ein Beutel ist, auf der Innenseite der Verpackung befindlich ist. Diese Polymerschicht ist dafür zuständig, dass die Verpackung durch Verschweißen dicht verschlossen werden kann. Die zweite Polymerschicht (B) der Folie muss mit sich selbst und mit entsprechend eingelegten Portelementen sicher und mit möglichst geringer Temperatur und Schweißzeit verschweißbar und trotzdem bei Temperaturen von mehr als 121°C hitzesterilisierbar sein. Eine niedrige Schweißtemperatur ist besonders wichtig, um die Folienstruktur möglichst wenig mit Gefügespannungen zu belasten. Demnach liegt die Schmelz- bzw. Erweichungstemperatur der zweiten Polymerschicht (B) im Allgemeinen oberhalb von 121°C, bevorzugt bei 122°C bis 135°C, besonders bevorzugt bei 124°C bis 130°C, in jedem Fall aber unterhalb der Schmelz- bzw. Erweichungstemperatur der ersten Polymerschicht (A).

Die zweite Polymerschicht (B) der erfindungsgemäßen Mehrschichtfolie enthält (oder besteht aus)
die Komponenten B1), B2) und B3) in folgenden Anteilen (jeweils bezogen auf (B)):
B1) 60 bis 85 Gew.-%, bevorzugt 65 bis 80 Gew.-%, besonders bevorzugt 72 bis 78 Gew.-%,
B2) 11 bis 30 Gew.-%, bevorzugt 15 bis 25 Gew.-%, besonders bevorzugt 17 bis 22 Gew.-%;
B3) 4 bis 15 Gew.-%, bevorzugt 4 bis 12 Gew.-%, besonders bevorzugt 5 bis 10 Gew.-%.

### Komponente B1)

Komponente B1) dient zur Verringerung der Delaminationsneigung zwischen der zweiten Polymerschicht (B) und der mittleren Polymerschicht (C).

Die homogene Zusammensetzung (= "Composite") (B1) besteht aus:
B11) 65 bis 85 Gew.-% - bezogen auf (B1) - Ethylen-Homopolymer, und
B12) 15 bis 35 Gew.-%, bevorzugt 20 bis 30 Gew.-%, - bezogen auf (B1) - mindestens eines, vorzugsweise eines, Ethylen-Copolymers, welches als Comonomer mindestens ein, vorzugsweise ein, alpha-Olefin mit 4 bis 12, bevorzugt 4 bis 8, besonders bevorzugt 4 bis 6 Kohlenstoffatomen, ganz besonders bevorzugt ausgewählt aus 1-Buten, 1-Penten, 1-Hexen und 4-Methyl-1-penten, vorzugsweise 1-Buten, enthält.

Die homogene Zusammensetzung (B1) weist eine Schmelztemperatur von > 125°C, vorzugsweise von 130 bis 135°C, und eine Dichte von 945 bis 960 kg/m³ auf.

Das Ethylen-Copolymer B12) enthält neben dem alpha-Olefin mit 4 bis 12 Kohlenstoffatomen keine weiteren Comonomere.

Der Anteil des alpha-Olefin-Comonomers - bezogen auf (B12) - beträgt im Allgemeinen 25 bis 40 Gew.-%, bevorzugt 30 bis 37 Gew.-%; der Ethylen-Anteil - bezogen auf (B12) - beträgt entsprechend 60 bis 75 Gew.-%, bevorzugt 63 bis 70 Gew.-%. Die Gewichtsanteile beziehen sich jeweils auf die einpolymerisierten Struktureinheiten der Monomere in das Ethylen-Copolymer (B12).

Bevorzugt ist Komponente B1) eine homogene Zusammensetzung bestehend aus:
B11) 70 bis 80 Gew.-% - bezogen auf (B1) - Ethylen-Homopolymer, und
B12) 20 bis 30 Gew.-% - bezogen auf (B1) - mindestens eines, vorzugsweise eines, Ethylen-Copolymers, welches als Comonomer mindestens ein, vorzugsweise ein, alpha-Olefin ausgewählt aus 1-Buten, 1-Penten, 1-Hexen und 4-Methyl-1-penten, vorzugsweise 1-Buten, enthält.

Besonders bevorzugt ist Komponente B1) eine homogene Zusammensetzung bestehend aus:
B11) 70 bis 80 Gew.-% - bezogen auf (B1) - Ethylen-Homopolymer, und
B12) 20 bis 30 Gew.-% - bezogen auf (B1) - Ethylen-1-Buten-Copolymer,
worin der Anteil 1-Buten - bezogen auf (B12) - 25 bis 40 Gew.-%, bevorzugt 30 bis 37 Gew.-%, beträgt.

Als Ethylen-Homopolymer B11) wird im Allgemeinen ein High-Density-Polyethylen (HDPE) eingesetzt, vorzugsweise ein HDPE mit einer Dichte von 950 bis 970 kg/m³.

Die Herstellung von HDPE ist dem Fachmann bekannt.

Ferner ist HDPE kommerziell erhältlich; HDPE mit einer Dichte von 950 bis 970 kg/m³ ist z.B. von Tosoh Co., Ltd. als Nipolon^{®} Hard oder von Borealis als Bormed^{®} HE2581-PH und Bormed HE7541-PH erhältlich.

Das Ethylen-Copolymer B12) wird üblicherweise durch Copolymerisation von Ethylen und dem alpha-Olefin-Comonomer mittels eines Metallocen-Katalysators hergestellt. Geeignete Metallocen-Katalysatoren sind organische Verbindungen eines Übergangsmetalls mit mehreren (Anzahl entsprechend der Wertigkeit des Übergangsmetalls) - mit dem Übergangsmetall koordinierten - Liganden, wovon mindestens ein Ligand ein Cyclopentadienylrest ist. Das Übergangsmetall ist bevorzugt ausgewählt aus der Gruppe bestehend aus Zr, Ti, Hf, V, Nb, Tn und Cr, besonders bevorzugt ist Zr oder Hf, ganz besonders bevorzugt Zr.

Bevorzugt ist ein Zr oder Hf-Metallocen-Katalysator mit zwei Cyclopentadienylresten.

Besonders bevorzugt ist der Metallocen-Katalysator Bis(n-butyl-cylopentadienyl)-zirkoniumdichlorid.

Zur Herstellung des Ethylen-Copolymers B12) werden die vorgenannten Metallocen-Katalysatoren als ionische Komplexverbindungen eingesetzt, die durch Umsetzung des Metallocen-Katalysators mit organisch modifizierter Tonerde erhalten werden können. Als Tonerde können alle üblichen Tonmaterialien eingesetzt werden, bevorzugt sind Hektorit, Smektit, und Montmorillonit.

Die organisch modifizierte Tonerde wird durch Umsetzung von Tonerde mit einem aliphatischen Salz erhalten. Beispiele für ein solches aliphatisches Salz sind N,N-Dimethyldecylaminhydrochlorid, N,N-Dimethyldodecylaminhydrochlorid, N,N-Dimethyltetradecylaminhydrochlorid, N,N-Dimethylhexadecylaminhydrochlorid, N,N-Dimethyl-Octadodecylamin-Hydrochlorid, N,N-Dimethyl-Behenylamin-Hydrochlorid, N,N-Dimethyl-Behenylamin-Hydrofluorid, N,N-Dimethyl-Behenylaminhydrobromid und N, N-Dimethyl-Behenylamin-Hydroiodid, bevorzugt ist N,N-Dimethyl-Behenylamin-Hydrochlorid.

Vorzugsweise wird zur Herstellung des Ethylen-Copolymers B12) noch eine Organoaluminiumverbindung, vorzugsweise Triisobutylaluminium, als Co-Katalysator eingesetzt.

Die Herstellung des Ethylen-Copolymers B12) kann in Gegenwart des Metallocen-Katalysators zum Beispiel durch ein Aufschlämmverfahren, ein Lösungsverfahren oder in der Gasphase erfolgen.

Die Herstellung von Ethylen-alpha-Olefin-Copolymeren mittels eines solchen Metallocen-Katalysators ist zum Beispiel in JP-A 2019/111805 und JP-A 2019/167430 beschrieben.

Das Ethylen-Copolymer B12) ist ein Ethylen-Copolymer hoher Dichte. Bevorzugt weist das Ethylen-Copolymer B12) eine Dichte von 945 bis 960 kg/m³ auf.

### Komponente B2)

Komponente B2) ist mindestens ein, vorzugsweise ein, Propylen-Terpolymer.

Der Begriff "Terpolymer" kennzeichnet ein Copolymer, welches aus drei verschiedenen Monomeren hergestellt ist.

Der Begriff "Propylen-Terpolymer" bezeichnet eine mit zwei zusätzlichen Co-Monomeren im Polymerisationsprozess modifizierte Polypropylen-Molekülkette. Bevorzugte zusätzliche Co-Monomere sind Ethylen und/oder mindestens ein C₄-C₁₂ α-Olefin, bevorzugt C₄-C₈ α-Olefin,besonders bevorzugt Ethylen und ein C₄-C₈ α-Olefin,ganz besonders bevorzugt Ethylen und 1-Buten.

Der Anteil von Ethylen beträgt vorzugsweise 1 bis 4 Gew.-%, der Anteil des mindestens einen C₄-C₈ α-Olefins, insbesondere 1-Buten, beträgt vorzugsweise 9 bis 12 Gew.-%, jeweils bezogen auf (B2). Die Gewichtsanteile beziehen sich jeweils auf die einpolymerisierten Struktureinheiten der Monomere in das Terpolymer (B2).

Ganz besonders bevorzugt Propylen-Terpolymer (B2) aufgebaut aus Struktureinheiten von Propylen, Ethylen und Butylen.

Die Herstellung von Propylen-Terpolymeren ist bekannt. Ferner sind geeignete Propylen-Terpolymere kommerziell erhältlich z.B. von Borealis, Österreich.

### Komponente B3)

Komponente B3) ist mindestens ein, vorzugsweise ein, Polyethylen-Elastomer, welches ein Copolymer von Ethylen mit mindestens einem, vorzugsweise einem, alpha-Olefin mit 4 bis 12, bevorzugt 7 bis 12, ganz besonders bevorzugt 8 Kohlenstoffatomen ist.

Ethylen/alpha-Olefin-Copolymere B3) haben bevorzugt eine Dichte im Bereich von 600 bis 950 kg/m³, besonders bevorzugt 750 bis 900 kg/m³.

Der Anteil der einpolymerisierten Struktureinheiten des alpha-Olefins - bezogen auf (B3) - beträgt mehr als 8 Gew.-%, vorzugsweise mehr als 10 Gew.-%, insbesondere 20 bis 30 Gew.-%.

Bevorzugt ist Komponente B3) ein Ethylen-1-Octen-Copolymer.

Geeignete Polyethylen-Elastomere sind kommerziell erhältlich z.B. von Dow Chemical Company, USA.

Vorzugsweise enthält (oder besteht aus) die zweite Polymerschicht (B) gemäß der Erfindung eine homogene Zusammensetzung (Komponente B1) bestehend aus B11) 70 bis 80 Gew.-% High-Density-Polyethylen und B12) 20 bis 30 Gew.-% Ethylen-1-Buten-Copolymer, ein Propylen-Terpolymer (Komponente B2) aufgebaut aus Struktureinheiten von Propylen, Ethylen und Butylen (Komponente B2), und ein Ethylen-1-Octen-Copolymer (Komponente B3).

Besonders bevorzugt enthält (oder besteht aus) die zweite Polymerschicht (B) gemäß der Erfindung 65 bis 80 Gew.-%, bevorzugt 72 bis 78 Gew.-% der homogenen Zusammensetzung (Komponente B1) bestehend aus B11) 70 bis 80 Gew.-% High-Density-Polyethylen und B12) 20 bis 30 Gew.-% Ethylen-1-Buten-Copolymer, 15 bis 25 Gew.-%, bevorzugt 17 bis 22 Gew.-% Propylen-Terpolymer (Komponente B2) aufgebaut aus Struktureinheiten von Propylen, Ethylen und Butylen (Komponente B2), und 4 bis 12 Gew.-%, bevorzugt 5 bis 10 Gew.-% Ethylen-1-Octen-Copolymer (Komponente B3).

Die Gewichtsangaben für die Komponenten B1), B2) und B3) der zweiten Polymerschicht (B) beziehen sich auf das Gesamtgewicht der zweiten Polymerschicht (B).

### Mittlere Polymerschicht (C)

Die mittlere Polymerschicht (C) hat den größten Massenanteil (mindestens 50 Gew.-%) der Mehrschichtfolie, bevorzugt 60 bis 95 Gew.-%, besonders bevorzugt 70 bis 90 Gew.-%, ganz besonders bevorzugt 75 bis 85 Gew.-% der gesamten Mehrschichtfolie und dient zur Verbesserung der Schlagzähigkeit der gesamten Struktur.

Die mittlere Polymerschicht (C) der erfindungsgemäßen Mehrschichtfolie enthält (oder besteht aus) die Komponenten C1), C2) und C3) in folgenden Anteilen (jeweils bezogen auf (C)):
C1) 61 bis 80 Gew.-%, bevorzugt 65 bis 75 Gew.-%,
C2) 15 bis 25 Gew.-%, bevorzugt 17 bis 22 Gew.-%,
C3) 5 bis 19 Gew.-%, bevorzugt 8 bis 17 Gew.-%.

### Komponente C1)

Komponente C1) ist mindestens ein, vorzugsweise ein, Propylen-Terpolymer. Propylen-Terpolymer C1) ist wie Komponente B2) definiert; auf die entsprechenden Ausführungen zu Komponente B2) wird verwiesen.

### Komponente C2)

Komponente C2) ist mindestens ein, bevorzugt ein, Styrol-Blockcopolymer (SBC) Elastomer.

Der Begriff "Styrol-Blockcopolymer Elastomer" bezeichnet synthetische thermoplastische Elastomere auf Basis von Styrol-Blockcopolymeren, die als Schlagzähmodifikator verwendet werden.

Das mindestens eine Styrol-Blockcopolymer (SBC) Elastomer B2) ist bevorzugt ausgewählt aus der Gruppe bestehend aus: Styrol-Ethylen-Butylen-Styrol-Blockcopolymer (SEBS), Styrol-Ethylen-Propylen-Styrol-Blockcopolymer (SEPS), Styrol-Ethylen-Ethylen-Propylen-Styrol-Blockcopolymer *(*SEEPS*),* Styrol-Isopren-Styrol-Blockcopolymer (SIS) und Styrol-Butadien-Styrol-Blockcopolymer (SBS), bevorzugt SEBS und SEPS, insbesondere SEBS.

Geeignete Styrol-Blockcopolymer (SBC) Elastomere C2) sind kommerziell erhältlich z.B. von Asahi Kasei, Japan.

Es ist auch möglich das Styrol-Blockcopolymer (SBC) Elastomer teilweise durch ein oder mehrere thermoplastische Elastomere auf Olefinbasis (TPE-O) zu ersetzen (Anteil TPE-O: maximal 45 Gew.-%, bevorzugt 20 bis 30 Gew.-%).

Bevorzugt ist Komponente C2) ein Styrol-Blockcopolymer (SBC) Elastomer, welches keine Anteile eines thermoplastischen Elastomere auf Olefinbasis enthält.

### Komponente C3)

Komponente C3) ist mindestens ein, vorzugsweise ein, Polyethylen-Elastomer, welches ein Copolymer von Ethylen mit mindestens einem alpha-Olefin mit 4 bis 12, bevorzugt 4 bis 8 Kohlenstoffatomen ist.

Ethylen/alpha-Olefin-Copolymere (C3) haben im Allgemeinen eine Dichte im Bereich von 440 bis 860 kg/m³.

Der Anteil der einpolymerisierten Struktureinheiten des alpha-Olefins - bezogen auf (C3) - beträgt mehr als 8 Gew.-%, vorzugsweise mehr als 10 Gew.-%, insbesondere 20 bis 30 Gew.-%.

Bevorzugt ist Komponente C3) ein Ethylen-1-Buten-Copolymer, ein Ethylen-1-Hexen-Copolymer oder ein Ethylen-1-Octen-Copolymer, besonders bevorzugt ein Ethylen-1-Octen-Copolymer.

Geeignete Polyethylen-Elastomere sind kommerziell erhältlich z.B. von Dow Chemical Company, USA.

Vorzugsweise enthält (oder besteht aus) die mittlere Polymerschicht (C) gemäß der Erfindung ein Propylen-Terpolymer (Komponente C1)) aufgebaut aus Struktureinheiten von Propylen, Ethylen und Butylen, ein Styrol-Ethylen-Butylen-Styrol-Blockcopolymer (SEBS) (Komponente C2)), und ein Ethylen-Octen-Copolymer (Komponente C3)).

Besonders bevorzugt enthält (oder besteht aus) die mittlere Polymerschicht (C) gemäß der Erfindung 65 bis 80 Gew.-%, bevorzugt 72 bis 78 Gew.-%, Propylen-Terpolymer (Komponente C1)) aufgebaut aus Struktureinheiten von Propylen, Ethylen und Butylen, 17 bis 22 Gew.-%, bevorzugt 19 bis 21 Gew.-%, Styrol-Ethylen-Butylen-Styrol-Blockcopolymer (SEBS) (Komponente C2)); und 4 bis 10 Gew.-%, bevorzugt 5 bis 8 Gew.-% Ethylen-Octen-Copolymer (Komponente C3)).

### Funktionsschicht (D)

Die hitzesterilisierbare Mehrschichtfolie gemäß der Erfindung kann ferner eine zusätzliche Funktionsschicht D) als Außenschicht umfassen, die sich benachbart zu der ersten Polymerschicht (A) auf der äußeren Seite von (A) (d.h. auf der anderen Seite von (A) gegenüberliegend zu der mit Polymerschicht (C)) befindet.

Funktionsschicht (D) bewirkt vorzugsweise, dass die hitzesterilisierbare Mehrschichtfolie, sowie daraus hergestellte Verpackungen wie medizinische Beutel oder daraus hergestellte Folienschläuche gasdicht und/oder wasserdicht sind.

Funktionsschicht D) enthält, bevorzugt besteht aus, mindestens ein, bevorzugt ein, Material ausgewählt aus der Gruppe bestehend aus: Ethylen-Vinylalkohol-Copolymeren, Polyvinylalkoholen, Polyamiden, flüssigkristalllinen Polymeren (LCP), aromatischen Polyestern, bevorzugt Terephthalsäure-Polyestern, besonders bevorzugt Polyethylentherephthalaten (PET), Siliziumoxid (SiOₓ), Aluminiumoxid (AlOₓ) und Polymeren auf Acyrylatbasis.

Bevorzugt besteht die Funktionsschicht (D) aus PET/SiOₓ.

Die Funktionsschicht (D) weist vorzugsweise eine Schichtdicke von 5 bis 25 µm, insbesondere von 10 bis 20 µm, auf.

Durch eine Funktionsschicht (D) aus PET/SiOₓ wird die Gasbarriere (z.B. die Sauerstoffbariere) der hitzesterilisierbaren Mehrschichtfolie gemäß der Erfindung signifikant verbessert, so dass die Folie auch gut für die Lagerung von sauerstoffempfindlichen Inhaltsstoffen geeignet ist.

Durch eine SiOₓ/PET-Funktionsschicht (D) kann die Sauerstoffbarriere (oxygen transmission rate (OTR)) der hitzesterilisierbaren Mehrschichtfolie gemäß der Erfindung um den Faktor 1000 auf OTR-Werte < 1 cm³/(m² x day) ASTM F1927 (23°C, 50% r.H.) abgesenkt werden.

### Mehrschichtfolie

Bevorzugt besteht die hitzesterilisierbare Mehrschichtfolie gemäß der Erfindung aus den Polymerschichten (A), (B) und (C).

Die Mehrschichtfolie kann in jeder der Polymerschichten (A), (B) und (C) übliche Additive und/oder Verarbeitungshilfsmittel, die für den Verwendungszweck der Mehrschichtfolie geeignet sind, in üblichen Mengen enthalten.

Bevorzugte Additive sind Antioxidantien und thermische Stabilisatoren (phosphitische und phenolische Stabilisatoren wie Irgafos^{®} 168, Irgafos P-EPQ , Irganox^{®} 1076 oder Irganox 1010), sowie Säurefänger wie z.B. DHT-4A^{®}, synthetischer Hydrotalcit (SHT) und Magnesiumoxid (MgO).

Bevorzugt enthält die hitzesterilisierbare Mehrschichtfolie aus den Polymerschichten (A), (B) und (C) gemäß der Erfindung mindestens ein Antioxidans, thermischen Stabilisator und/oder Säurefänger, bevorzugt in einer Gesamtmenge von < 3000 ppm, bezogen auf die gesamte Mehrschichtfolie.

Bevorzugt haften die Polymerschichten (A), (B) und (C) aneinander, ohne dass ein Haftvermittler eingesetzt wird, d.h. die erfindungsgemäße Mehrschichtfolie aus den Polymerschichten (A), (B) und (C) enthält bevorzugt keinen Haftvermittler. Ferner enthält bevorzugt zumindest die zweite Polymerschicht (B) keine weiteren Additive und/oder Verarbeitungshilfsmittel (z.B. Modifikatoren oder Weichmacher, wie z.B. Mineralöl), wobei ganz besonders bevorzugt keine der Polymerschichten (A), (B), und (C) weitere Additive und/oder Verarbeitungshilfsmittel, zusätzlich zu den vorstehend genannten Additiven, enthält. Demnach findet keine oder kaum eine Beeinflussung des Medikaments oder der medizinischen Lösung durch die erfindungsgemäße Mehrschichtfolie als Packmittel während der Sterilisation und der Lagerung statt.

Im Fall einer erfindungsgemäßen Mehrschichtfolie bestehend aus den Polymerschichten (A), (B), (C) und der Funktionsschicht (D) enthält die Mehrschichtfolie im Allgemeinen zusätzlich zu den vorstehend als bevorzugt genannten Additiven einen Haftvermittler bzw. Haftkleber.

Die Schichtdicke der ersten Polymerschicht (A) beträgt im Allgemeinen 5 bis 15 Gew.-%, bevorzugt 7 bis 13 Gew.-%, besonders bevorzugt 7,5 bis 10 Gew.-%, der gesamten Folienstärke der erfindungsgemäßen Mehrschichtfolie.

Die Schichtdicke der zweiten Polymerschicht (B) beträgt im Allgemeinen 5 bis 15 Gew.-%, bevorzugt 7 bis 13 Gew.-%, besonders bevorzugt 7,5 bis 10 Gew.-%, der gesamten Folienstärke der erfindungsgemäßen Mehrschichtfolie.

Die mittlere Polymerschicht (C) hat den größten Anteil (bevorzugt mindestens 70 Gew.-% der gesamten Folienstärke) der erfindungsgemäßen Mehrschichtfolie und dient zur Verbesserung der Schlagzähigkeit der gesamten Struktur.

Falls vorhanden, beträgt die Schichtdicke der optionalen Funktionsschicht (D) bevorzugt 2,5 bis 12,5 Gew.-%, besonders bevorzugt 5 bis 10 Gew.-%, der gesamten Folienstärke der erfindungsgemäßen Mehrschichtfolie.

Die gesamte Folienstärke (= Dicke) der erfindungsgemäßen Mehrschichtfolie beträgt bevorzugt 50 bis 500 µm, besonders bevorzugt 100 bis 400 µm, ganz besonders bevorzugt 150 bis 300 µm.

Die gesamte Folienstärke bzw. Dicke einer erfindungsgemäßen Mehrschichtfolie bestehend aus den Polymerschichten (A), (B) und (C) beträgt bevorzugt 50 bis 500 µm, besonders bevorzugt 100 bis 400 µm, ganz besonders bevorzugt 150 bis 300 µm.

Besonders bevorzugt ist eine erfindungsgemäße Mehrschichtfolie, die aus den Polymerschichten (A), (B) und (C) besteht, und dadurch gekennzeichnet ist, dass die gesamte Folienstärke der Mehrschichtfolie 50 bis 500 µm, besonders bevorzugt 100 bis 400 µm, beträgt, und - jeweils bezogen auf die gesamte Folienstärke der Mehrschichtfolie
die Schichtdicke der ersten Polymerschicht (A) 5 bis 15 Gew.-%, bevorzugt 7 bis 13 Gew.-%,
die Schichtdicke der zweiten Polymerschicht (B) 5 bis 15 Gew.-%, bevorzugt 7 bis 13 Gew.-%; und
die Schichtdicke der mittleren Polymerschicht (C) 70 bis 85 Gew.-%, bevorzugt 74 bis 80 Gew.-%; beträgt; und
die Anteile von (A), (B) und (C) jeweils zusammen 100 Gew.-% ergeben.

### Verfahren zur Herstellung der Mehrschichtfolie

Ein weiterer Gegenstand der Erfindung ist ein Verfahren zur Herstellung der erfindungsgemäßen Mehrschichtfolie, wobei die erste Polymerschicht (A), die mittlere Polymerschicht (C), und die zweite Polymerschicht (B) coextrudiert werden.

Bei der Coextrusion werden die Kunststoffschmelzen der Polymerschichten (A), (B) und (C) vor dem Verlassen der Profildüse eines Extruders zu der erfindungsgemäßen Mehrschichtfolie zusammengeführt.

Oftmals ist der Extrusionsprozess zweistufig. In einem ersten Schritt werden die für die einzelnen Polymerschichten verwendeten Materialien in Extrudern, vorzugsweise parallelen Doppelschneckenextrudern (Compoundern), Heiz-Kühl-Mischern oder Pelletierpressen gemischt und kompaktiert. Dann werden die Kunststoffschmelzen der Polymerschichten (A), (B) und (C) in einem anderen, direkt gekoppelten oder räumlich und zeitlich getrennten Extruder vor dem Verlassen der Profildüse zu der erfindungsgemäßen Mehrschichtfolie zusammengeführt.

Bevorzugt wird die durch das erfindungsgemäße Verfahren erhaltene Mehrschichtfolie mit Wasser schockgekühlt.

Durch die Coextrusion kann die erfindungsgemäße Mehrschichtfolie in Form einer flachen Folie (Flachfolienverfahren, z.B. bei Verwendung einer Breitschlitzdüse) oder eines Folienschlauches (Blasfolienverfahren, z.B. Flutung des Innenraums des Folienschlauches mit - vorzugsweise steril gefilterter - Luft) erhalten werden, wobei im Fall eines Folienschlauches die Außenseite aus der ersten Polymerschicht (A) und die Innenseite aus der zweiten Polymerschicht (B) besteht.

Auf die durch das erfindungsgemäße Verfahren erhaltene Mehrschichtfolie kann in einem weiteren Verfahrensschritt die optionale Funktionsschicht (D) - z.B. durch Heißlaminieren oder bevorzugt durch Kaschieren - aufgebracht werden.

Gemäß einer besonderen Ausführungsform zur Herstellung eines kaschierten Mehrschichtfolienschlauches gemäß der Erfindung umfasst das erfindungsgemäße Verfahren die folgenden Schritte:
(a`) Herstellen eines Folienschlauches aus der erfindungsgemäßen Mehrschichtfolie durch Coextrusion der ersten Polymerschicht (A), der mittleren Polymerschicht (C), und der zweiten Polymerschicht (B), wobei der Innenraum des Folienschlauches mit - vorzugsweise steril gefilterter - Luft geflutet wird;
(b') gegebenenfalls Abkühlen des in Verfahrensschritt (a') hergestellten Folienschlauches;
(c') Beschichten des gegebenenfalls abgekühlten Folienschlauches mit einer Haftkleberadhäsivschicht auf mindestens einer Seite (erste Polymerschicht (A)) des Folienschlauches;
(d') gegebenenfalls Trocknen des mit der Haftkleberadhäsivschicht versehenen Folienschlauches;
(e') Kaschieren der mindestens einen mit der Haftkleberadhäsivschicht beschichteten Seite (erste Polymerschicht (A)) des Folienschlauches mit einer Funktionsschicht (D), insbesondere einer SiOx/PET-Funktionsschicht;
(f) gegebenenfalls Trocknen bzw. Aushärten des kaschierten Folienschlauches.

Gemäß dem erfindungsgemäßen Verfahren der vorgenannten Ausführungsform haften die beiden parallelen innenliegenden Seiten (zweite Polymerschichten (B)) des Folienschlauches nach der Schmelz- bzw. Coextrusion des Folienschlauches vorzugsweise unmittelbar aufeinander, so dass eine Beschichtung der Außenseiten (erste Polymerschichten (A)) des Folienschlauches bei einem geschlossenen Innenraum des Folienschlauches möglich ist. Der dabei geschlossene Innenraum, der bei einer späteren Verwendung des resultierenden Folienschlauches aufgebläht wird, ist damit im Wesentlichen partikelfrei.

Bevorzugt wird bei dem erfindungsgemäßen Verfahren der vorgenannten Ausführungsform der Innenraum des Folienschlauches mit steril gefilterter Luft geflutet, so dass ein partikelarmer, kaschierter Mehrschichtfolienschlauch erhalten wird, der für medizinische Zwecke besonders gut geeignet ist.

Besonders bevorzugt wird zur Herstellung eines partikelarmen, kaschierten Mehrschichtfolienschlauches das vorgenannte erfindungsgemäße Verfahren in einem Reinraum durchgeführt.

In dem erfindungsgemäßen Verfahren gemäß obiger Ausführungsform wird vorzugsweise ein Haftvermittler verwendet, der eine vollständige Aushärtung bei Raumtemperatur nach ungefähr 2 Wochen, vorzugsweise 1 Woche, ermöglicht. In einer Wärmekammer kann die Aushärtung bei erhöhter Temperatur, vorzugsweise 30°C oder mehr, oftmals 40°C bis 60 °C, auch schneller erfolgen.

Geeignete Haftvermittler (Haftkleber, Adhäsivkleber oder Kaschierkleber) sind beispielsweise Isocyanate, Polyurethane, Poly(ethylacrylat/methacrylsäureester), Reinacrylatcopolymerisate, Vinylester-/Acrylat-Copolymerisate oder anorganischeorganische Hybridpolymere.

Bevorzugte Haftvermittler sind Zweikomponentensysteme, die lösemittelhaltig oder lösemittelfrei, und silanhaltig oder silanfrei sein können und optional zur Beschleunigung der Aushärtung mit einem zusätzlichen "Catalyst" verwendet werden können.

Geeignete lösemittelhaltige Zweikomponentensysteme sind zum Beispiel Polyurethan-Adhäsivkleber u.a. kommerziell erhältliche Systeme wie
- Dow ADCOTE^{™} 675A + ADCOTE^{™} 675C coreactant;
- Dow ADCOTE 811A + ADCOTE 811B coreactant;
- Dow ADCOTE E735A-75 + ADCOTE^{™} E735C2 coreactant:
- MORCHEM PS 241 AE + CS-97 coreactant,
- Henkel Loctite Liofol LA2798 + Henkel Loctite Liofol LA7371;
- Henkel LOCTITE HY 4070 2K-Hybridklebstoff.

Die vorgenannten Systeme können optional mit "Catalysts" eingesetzt werden u.a. solchen wie Dow Catalyst 9L10 (Polyisocyanat), Dow Catalyst 9L200 und Dow Catalyst F Adcote 40-3E, die kommerziell erhältlich sind.

Geeignete lösemittelfreie Zweikomponentensysteme sind zum Beispiel Polyurethan-Adhäsivkleber u.a. kommerziell erhältliche Systeme wie
- Dow MOR-FREE^{™} L 75-720 Adhesive + CR 88-720 or CR 88-721 or MOR-FREE^{™} C 79 S coreactant
- Dow MOR-FREE^{™} 203A Adhesive + MOR-FREE^{™} 200C coreactant
- Dow MOR-FREE^{™} L705 Adhesive + MOR-FREE^{™} C 79 or MOR-FREE^{™} C-102 coreactant.

Alternativ können auch Einkomponentensysteme,als Haftvermittler eingesetzt werden, die lösemittelhaltig oder lösemittelfrei, und silanhaltig oder silanfrei sein können und optional zur Beschleunigung der Aushärtung mit einem zusätzlichen "Catalyst" verwendet werden können.

Geeignete lösemittelfreie Einkomponentensysteme sind zum Beispiel Dow MOR-FREE^{™} ELM 415A (Polyurethan-Adhäsivkleber) oder SENOBOND^{®}-WB-FOLIENKASCHIERKLEBER FP NDC 375224, die kommerziell erhältlich sind.

Besonders bevorzugt wird der Adhäsivkleber so gewählt, dass er die Anforderungen von Pharmakopoe Limits, beispielsweise hinsichtlich von Migrationseigenschaften einhält und vorzugsweise frei von organischen Lösungsmitteln ist.

Die Adhäsivschicht kann - je nach Verfahrensweise oder gewünschter Beschichtung - einseitig oder beidseitig auf den durch Coextrusion hergestellten Folienschlauch aufgetragen werden. Dieses kann beispielsweise durch Aufspritzen oder Aufrakeln erfolgen. Geeignet ist auch die Verwendung von wässrigen Lösungen der entsprechenden Adhäsivmittel.

Nach dem Auftragen dieser Adhäsivschichten kann der resultierende Folienschlauch gegebenenfalls getrocknet werden. Beispielsweise kann, wenn die Auftragung des Adhäsivs unter Verwendung von Wasser erfolgt, das Trocknen unter Verdunstung des Wassers erfolgen.

Die Schichtdicken der Haftkleberadhäsivschichten liegen vorzugsweise im Bereich von 3 bis 10 µm.

Erfindungsgegenstand ist außerdem die Verwendung der erfindungsgemäßen Mehrschichtfolie zur Herstellung einer medizinischen Verpackung, vorzugsweise eines medizinischen Beutels.

Auch die Verwendung der erfindungsgemäßen medizinischen Verpackung, bevorzugt als Behälter für mindestens ein Medikament ist Gegenstand der Erfindung.

Die erfindungsgemäße medizinische Verpackung ist insbesondere als Behälter für mindestens ein Medikament geeignet, wobei aufgrund der Kombination von einer

Polypropylen-basierten Außen- und Mittelschicht (Polymerschichten (A) und (C)) mit einer Polyethylen-basierten Innenschicht (Polymerschicht (B)), eine sehr gute Verarbeitbarkeit auf allen gängigen Beutelschweißmaschinen gewährleistet ist und gleichzeitig das Polyethylen der Innenschicht eine besonders geringe Adsorptionsneigung gegenüber medizinischen Wirkstoffen aufweist.

In einer bevorzugten Ausführungsform ist die erfindungsgemäße Verpackung in Kammern unterteilt, so dass sie als Behälter für mehrere Medikamente gleichzeitig genutzt werden kann. Dies ist beispielsweise bei solchen Medikamentenkombinationen von Relevanz, die zusammen verabreicht werden müssen, jedoch in Kombination nicht über längere Zeiträume stabil sind, oder bei festen Medikamenten, die in Form einer Lösung oder Suspension verabreicht werden, jedoch in der Lösung oder in der Suspension nicht über längere Zeiträume stabil sind. Durch getrennte Kammern lassen sich die Bestandteile der endgültigen Verabreichungsform getrennt aufbewahren und kurz vor der Verabreichung durch Öffnen der Trennstellen miteinander vermischen.

Ein Verfahren zur Herstellung einer erfindungsgemäßen medizinischen Verpackung, bevorzugt eines Beutels, umfasst die Schritte:
a) Bereitstellen mindestens einer erfindungsgemäßen hitzesterilisierbaren Mehrschichtfolie;
b) gegebenenfalls Bereitstellen eines oder mehrerer Portelemente und/oder Schläuche;
c) Formen einer medizinischen Verpackung, bevorzugt eines Beutels, aus der mindestens einen hitzesterilisierbaren Mehrschichtfolie, so dass die zweite Polymerschicht (B) die innere Fläche der medizinischen Verpackung, bevorzugt des Beutels, und die erste Polymerschicht (A) die äußere Fläche der medizinischen Verpackung, bevorzugt des Beutels, bildet;
d) gegebenenfalls Positionieren der Portelemente und/oder Schläuche zwischen den inneren Flächen an den Konturen der medizinischen Verpackung, bevorzugt des Beutels;
e) In-Kontakt-Bringen der inneren Flächen an den Konturen der medizinischen Verpackung, bevorzugt des Beutels, miteinander und mit gegebenenfalls dazwischen positionierten Portelementen und/oder Schläuchen;
f) Verschweißen der inneren Flächen an den Konturen der medizinischen Verpackung, bevorzugt des Beutels, miteinander und mit gegebenenfalls dazwischen positionierten Portelementen und/oder Schläuchen.

In Schritt a) wird die erfindungsgemäße Mehrschichtfolie vorzugsweise in Form einer Flachfolie oder einer Schlauchfolie bereitgestellt. Abhängig von der bereitgestellten Form der Folie kann sich das weitere Verfahren in bestimmten Einzelheiten unterscheiden. Der erhaltene partikelarme Folienschlauch kann durch zusätzliche Verfahrensschritte mit einer Funktionsschicht (D) z.B. einer SiOx/PET-Funktionsschicht kaschiert werden.

Abhängig von der Anwendung der erfindungsgemäßen medizinischen Verpackung, bevorzugt des Beutels, können bei dem Verfahren nach der Bereitstellung der erfindungsgemäßen Mehrschichtfolie optional in Schritt b) zusätzliche Elemente, wie beispielsweise Portelemente und/oder Schläuche bereitgestellt werden. Die Bereitstellung dieser Elemente ist beispielsweise dann sinnvoll, wenn die erfindungsgemäße medizinische Verpackung, bevorzugt der Beutel, als fester Bestandteil einer medizinischen Vorrichtung eingesetzt werden soll oder an eine medizinische Vorrichtung angeschlossen werden soll. Das Weglassen des Schritts b) kann beispielsweise sinnvoll sein, wenn die medizinische Verpackung, bevorzugt der Beutel, lediglich der Aufbewahrung eines Medikaments dient und zur Entnahme des Medikaments beispielsweise durch Aufreißen oder Durchstechen mit einer Kanüle beschädigt wird.

In Schritt c) wird die bereitgestellte erfindungsgemäße Mehrschichtfolie in die Form einer medizinischen Verpackung, bevorzugt eines Beutels, gebracht. Wenn in Schritt a) eine Schlauchfolie bereitgestellt wurde, kann das Formen der medizinischen Verpackung, bevorzugt des Beutels, beispielsweise nur das Zuschneiden der Schlauchfolie auf die gewünschte Länge beinhalten, da bereits die zweite Polymerschicht (B) die innere Fläche der Schlauchfolie und die erste Polymerschicht (A) die äußere Fläche der Schlauchfolie bildet. Wenn in Schritt a) eine Flachfolie bereitgestellt wurde, kann in Schritt c) die medizinische Verpackung, bevorzugt der Beutel, beispielsweise aus einem Stück Mehrschichtfolie geformt werden, indem dieses Stück in eine spiegelsymmetrische Form zugeschnitten wird und entlang der Spiegelachse umgeklappt wird, sodass die Ränder der Folie sich deckungsgleich aufeinander legen, mit der zweiten Polymerschicht (B) auf der Innenseite. Alternativ kann die erfindungsgemäße medizinische Verpackung, bevorzugt der Beutel, beispielsweise aus zwei Stücken Flachfolie geformt werden, indem die zwei Stücke zueinander spiegelsymmetrisch zugeschnitten werden und deckungsgleich aufeinandergelegt werden, mit der zweiten Polymerschicht (B) auf der Innenseite. Bei dem Zuschneiden sind rechteckige Formen besonders bevorzugt, da hierdurch der Materialverlust am geringsten und die Verarbeitbarkeit am einfachsten ist. Allerdings sind auch andere Formen möglich, sodass beispielsweise eine medizinische Verpackung, bevorzugt ein Beutel, mit einer ästhetischen Form hergestellt werden kann, die für Kinder ansprechend ist und diese von der eigentlichen Verabreichung eines Medikaments ablenkt.

Abhängig davon, ob in Schritt b) zusätzliche Elemente wie Portelemente und/oder Schläuche bereitgestellt wurden, können diese Elemente in Schritt d) zwischen den inneren Flächen an den Konturen der geformten medizinischen Verpackung, bevorzugt des Beutels, positioniert werden. Im Fall einer Schlauchfolie ist hiermit das Einlegen der zusätzlichen Elemente in die Öffnungen der Schlauchfolie gemeint. Hierbei können die Elemente nur auf zwei einander gegenüberliegenden Seiten der medizinischen Verpackung, bevorzugt des Beutels, positioniert werden. Im Fall einer Flachfolie ist das Einlegen der zusätzlichen Elemente zwischen die in Schritt c) deckungsgleich aufeinander gelegten Ränder des einen oder der mehreren Flachfolienstücke gemeint. Hierbei können die Elemente an beliebigen Stellen entlang der Ränder positioniert werden, bevorzugt höchstens an zwei gegenüberliegenden Rändern.

In Schritt e) werden die inneren Flächen der geformten medizinischen Verpackung, bevorzugt des Beutels, an dessen Konturen miteinander und mit den gegebenenfalls zwischen den inneren Flächen befindlichen zusätzlichen Elementen in Kontakt gebracht, damit diese in Schritt f) durch Zuführen von Hitze und gegebenenfalls mechanischem Druck zusammen verschweißt werden können. Bei dem Verschweißen ist die Temperatur bevorzugt so gewählt, dass sie über dem Schmelz- bzw. Erweichungspunkt der zweiten Polymerschicht (B), jedoch unter dem Schmelz- bzw. Erweichungspunkt der ersten Polymerschicht (A) liegt. Hierdurch kann gewährleistet werden, dass die zweite Polymerschicht (B) an den Konturen der medizinischen Verpackung, bevorzugt des Beutels, schmilzt und diesen dadurch fest und fluiddicht verschließt, während die erste Polymerschicht (A) ihre Form behält und dadurch die Stabilität der medizinischen Verpackung, bevorzugt des Beutels, erhält.

Ein wichtiges Kriterium für den Einsatz der erfindungsgemäßen Mehrschichtfolie als Primärpackmittel für medizinische Lösungen ist die Sperrwirkung gegen Flüssigkeitsverlust. Durch einen solchen Flüssigkeitsverlust entsteht eine Aufkonzentrierung der Lösungs-Wirkstoffe, der bestimmte Werte nicht überschreiten darf. Der Flüssigkeitsverlust während der Lagerung entscheidet unter anderem über die Haltbarkeitsdauer des Produktes. Die Formulierung der erfindungsgemäßen Mehrschichtfolie ist so gewählt, dass bei guter Schlagzähigkeit eine sehr gute Wasserdampf-Barriere erzielt wird.

Die erfindungsgemäße Mehrschichtfolie zeichnet sich dadurch aus, dass sie hitzesterilisierbar, schlagzäh und flexibel (ohne Weichmacher) ist und mit einem thermisch dauerbeheizten Schweißverfahren auch mit Portelementen sicher verschweißbar ist, und dass sie ferner eine gute Haftung zwischen den einzelnen Schichten besitzt und dazu keinen Haftvermittler benötigt und medizinische Lösungen oder Medikamente nicht oder zumindest kaum beeinflusst werden.

Mit einer zusätzlichen Funktionsschicht (D), insbesondere einer SiOx/PET-Funktionsschicht, versehene erfindungsgemäße Mehrschichtfolien weisen eine deutlich verbesserte Gasbarriere auf, die auch eine Lagerung von sauerstoffempfindlichen Inhaltsstoffen erlaubt.

Nachfolgend wird die Erfindung anhand von Beispielen näher erläutert, ohne sie dadurch einzuschränken.

### Beispiel 1:

Erste Polymerschicht (A):
- 97 Gew.-% Moplen^{®} HP525J von Lyondell Basell Corp., USA/ Propylen-Homopolymer
- 3 Gew.-% Tuftec^{®} H1062 von Asahi Kasei, Japan/ Styrol-Ethylen/Butylen-Blockcopolymer

Die genannte Formulierung wurde in einem separaten Compoundierschritt im Schmelzezustand homogen gemischt, extrudiert und für den weiteren Einsatz granuliert.

### Zweite Polymerschicht (B):

B1) 75 Gew.-% Tosoh FY-13 von Tosoh Corp., Japan // Composite aus Ethylen-Homopolymer (70 bis 80 Gew.-%) und Ethylen-1-Buten-Copolymer (20 bis 30 Gew.-%)/ Dichte: 950 kg/m³, Tₘ: 128°C
B2) 20 Gew.-% Bormed^{®} TD109CF von Borealis, Österreich/ Propylen-Terpolymer
B3) 5 Gew.-% Engage^{®} 8003 von Dow Chemical Company, USA/ Ethylen-Octen-Polyolefin Elastomer

Die genannte Formulierung wurde in einem separaten Compoundierschritt im Schmelzezustand homogen gemischt, extrudiert und für den weiteren Einsatz granuliert.

Mittlere Polymerschicht (C):
C1) 70 Gew.-% Bormed^{®} TD109CF von Borealis, Österreich/ Propylen-Terpolymer
C2) 20 Gew.-% Tuftec^{®} H1062 von Asahi Kasei, Japan/ Styrol-Ethylen/Butylen-Blockcopolymer
C3) 10 Gew.-% Engage^{®} 8003 von Dow Chemical Company, USA/ Ethylen-Octen-Polyolefin Elastomer

Die genannte Formulierung wurde in einem separaten Compoundierschritt im Schmelzezustand homogen gemischt, extrudiert und für den weiteren Einsatz granuliert.

Die Schmelzen aus den granulierten Compounds der ersten Polymerschicht (A), der mittleren Polymerschicht (C) und der zweiten Polymerschicht (B) wurden mit für Polypropylen üblichen Prozessparametern auf einer Blasfolienanlage mit Wasserkühlung coextrudiert und eine Mehrschicht-Folie in Form eines Folienschlauchs, dessen Innenraum mit steril gefilterter Luft geflutet wurde, wurde erhalten.

Die Folie wurde mit einer Gesamtdicke von 200 µm gefertigt, wobei die erste Polymerschicht (A) und zweite Polymerschicht (B) jeweils eine Dicke von 15 µm und die mittlere Polymerschicht (C) eine Dicke von 170 µm aufweist.

Die hergestellte Folie ist heißdampfsterilisierbar und bereits mit 125°C temperierten Schweißwerkzeugen fest verschweißbar.

### Beispiel 2:

Ein gemäß Beispiel 1 hergestellter Folienschlauch wurde zusätzlich beidseitig mit einer Funktionsschicht (D) aus SiOx/PET (Techbarrier T von Mitsubishi) in einer Schichtdicke von jeweils 15 µm versehen.

Zunächst wurde auf den flachgelegten Folienschlauch beidseitig ein Zweikomponentenkleber (Dow ADCOTE 811A + ADCOTE 811B coreactant, sowie Dow Catalyst 9L10, erhältlich von Dow Chemical) aufgetragen und dann wurde der mit dem Kleber versehene Folienschlauch beidseitig mit der Funktionsschicht kaschiert.

Die hergestellte Folie ist heißdampfsterilisierbar und bereits mit 125°C temperierten Schweißwerkzeugen fest verschweißbar.

### Beispiel 3 (Vergleichsbeispiel gemäß DE 10361851 A1, Beispiel 1)

### Erste Polymerschicht (A):

- 97 Gew.-% Moplen^{®} HP525J von LyondellBasell Corp., USA/ Polypropylen-Homopolymer
- 3 Gew.-% Tuftec^{®} H1062 von Asahi Kasei, Japan/ Styrol-Ethylen/Butylen-Blockcopolymer

Die genannte Formulierung wurde in einem separaten Compoundierschritt im Schmelzezustand gemischt und für den weiteren Einsatz granuliert.

### Zweite Polymerschicht (B):

- 85 Gew.-% Bormed^{®} TD109CF von Borealis, Österreich/ Propylen-Terpolymer
- 15 Gew.-% Tuftec^{®} H1062 von Asahi Kasei, Japan/ Styrol-Ethylen/Butylen-Blockcopolymer

Die genannte Formulierung wurde in einem separaten Compoundierschritt im Schmelzezustand gemischt und für den weiteren Einsatz granuliert.

### Mittlere Polymerschicht (C):

- 75 Gew.-% Bormed^{®} TD109CF von Borealis, Österreich/ Propylen-Terpolymer
- 20 Gew.-% Tuftec^{®} H1062 von Asahi Kasei, Japan/ Styrol-Ethylen/Butylen-Blockcopolymer
- 5 Gew.-% Engage^{®} 8003, Dow Chemical Company, USA/ Ethylen-Octen-Polyolefin Elastomer

Die genannte Formulierung wurde in einem separaten Compoundierschritt im Schmelzezustand gemischt und für den weiteren Einsatz granuliert.

Die Folie wurde mit für Polypropylen üblichen Prozessparametern auf einer Blasfolienanlage mit Wasserkühlung coextrudiert.

Die Folie wurde mit einer Gesamtdicke von 200 µm gefertigt, wobei die erste Polymerschicht (A) und zweite Polymerschicht (B) jeweils eine Dicke von 15 µm und die mittlere Polymerschicht (C) eine Dicke von 170 µm aufweist. Die hergestellte Folie ist heißdampfsterilisierbar und mit 125°C temperierten Schweißwerkzeugen fest verschweißbar.

### Beispiel 4 (nicht erfindungsgemäß, Vergleich)

### Erste Polymerschicht (A):

- 97 Gew.-% Moplen^{®} HP525J von LyondellBasell Corp., USA/ Polypropylen-Homopolymer
- 3 Gew.-% Tuftec^{®} H1062 von Asahi Kasei, Japan/ Styrol-Ethylen/Butylen-Blockcopolymer

Die genannte Formulierung wurde in einem separaten Compoundierschritt im Schmelzezustand homogen gemischt, extrudiert und für den weiteren Einsatz granuliert.

### Zweite Polymerschicht (B):

- 75 Gew.-% Bormed^{®} LE6600-PH von Borealis, Östereich/ low density polyethylene (LDPE)
- 20 Gew.-% Bormed^{®} TD109CF von Borealis, Österreich/ Propylen-Terpolymer
- 5 Gew.-% Engage^{®} 8003 von Dow Chemical Company, USA/ Ethylen-Octen-Polyolefin Elastomer

Die genannte Formulierung wurde in einem separaten Compoundierschritt im Schmelzezustand homogen gemischt, extrudiert und für den weiteren Einsatz granuliert.

### Mittlere Polymerschicht (C):

- 70 Gew.-% Bormed^{®} TD109CF von Borealis, Österreich/ Propylen-Terpolymer
- 20 Gew.-% Tuftec^{®} H1062 von Asahi Kasei, Japan/ Styrol-Ethylen/Butylen-Blockcopolymer
- 10 Gew.-% Engage^{®} 8003, Dow Chemical Company, USA/ Ethylen-Octen-Polyolefin Elastomer

Die genannte Formulierung wurde in einem separaten Compoundierschritt im Schmelzezustand homogen gemischt, extrudiert und für den weiteren Einsatz granuliert.

Die granulierten Compounds der Polymerschichten (A), (B) und (C) wurden mit für Polypropylen üblichen Prozessparametern auf einer Blasfolienanlage mit Wasserkühlung coextrudiert und eine Mehrschicht-Folie in Form eines Folienschlauchs wurde erhalten.

Die Folie wurde mit einer Gesamtdicke von 200 µm gefertigt, wobei die erste Polymerschicht (A) und zweite Polymerschicht (B) jeweils eine Dicke von 15 µm und die mittlere Polymerschicht (C) eine Dicke von 170 µm aufweist.

### Untersuchung der Schweißnahtfestigkeit (SNF) von Folien der Beispiele 1 bis 4

1.
   (a) Herstellung von verschweißten Proben mit einem Folienschweißgerät (IST Med von Kopp) aus 2 übereinanderliegenden Folien (je 20 x15 cm); Schweißbereich (Folienrand) -15 mm.
      Schweißparameter: Druck: 2-3 bar, Zeit: 1-2 Sekunden, Spalt: 320µm.
   (b) anschließend, Ausstanzen von 15 X 80 mm breiten Probestreifen mit verschweißtem Rand (= unsterilisierte Proben).
2. Heißdampfsterilisation bei 121 °C (Autoklav WEBECO Typ A 35, 2 bar, 20 Minuten) einiger der erhaltenen Probestreifen mit verschweißtem Rand (= heißdampfsterilisierte Proben). Um das Verblocken der Folie zu verhindern, werden die nicht verschweißten Bereiche der Probestreifen vorher geöffnet und Papier dazwischen gelegt.
3. Bestimmung der Schweißnahtfestigkeit durch Einspannen der abgekühlten Probestreifen mit verschweißtem Rand in eine Zugprüfmaschine (Zwick iLine 500N der Firma Zwick) und Ziehen der Probestreifen mit einem Zug von 400 mm/min.

Die Ergebnisse zur Bewertung der Schweißnaht werden in N/15mm angegeben.

Die Schweißnahtfestigkeit der Mehrschichtfolie sollte mehr als 25N/15 mm, bevorzugt mehr als 30N/15mm, betragen. Falls die Schweißnahtfestigkeit der Mehrschichtfolie 10 bis 20 N/15mm beträgt, handelt es sich um eine Peelnaht.

Figur 1 zeigt Schweißkurven unsterilisierter Proben gemäß den Beispielen 1 bis 4. Die x-Achse gibt die Schweißtemperatur [°C] an; die y-Achse die Zugfestigkeit [N/15mm].

Figur 2 zeigt Schweißkurven heißdampfsterilisierter Proben gemäß den Beispielen 1 bis 4. Die x-Achse gibt die Schweißtemperatur [°C] an; die y-Achse die Zugfestigkeit [N/15mm].

In den Figuren sind die einzelnen Beispiele durch verschiedene Linienstruktur gekennzeichnet.

### Ergebnisse:

### (a) Unsterilisierte Proben

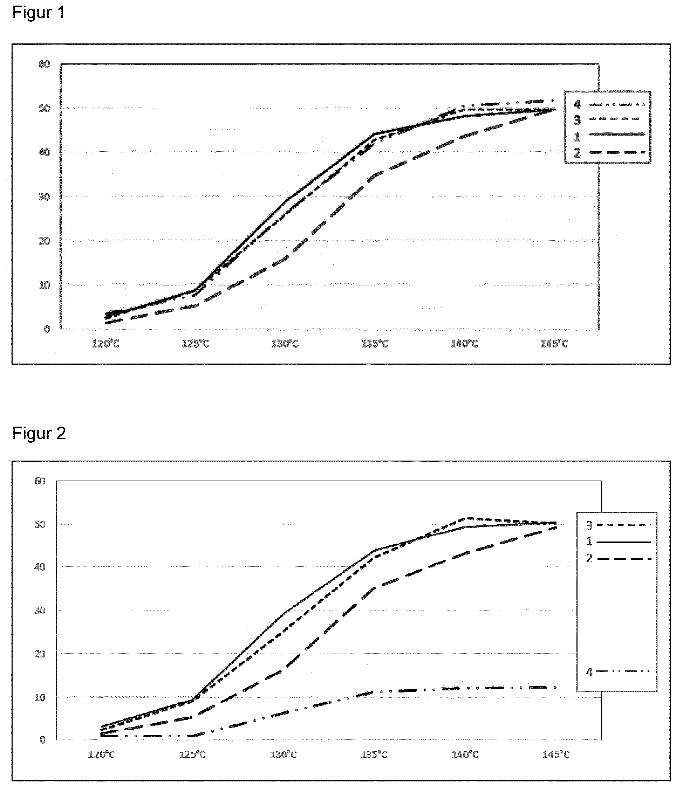

Die Schweißkurven (s. Figur 1) der Folien der Beispiele 1, 3 und 4 weisen bei ca. 130°C eine SNF von >30N/15mm auf; die Folie von Beispiel 2 mit der PET/SiOX-Funktionsschicht beginnt etwa 5°C höher zu verschweißen (SNF >30N/15mm), was auf Grund der höheren Temperaturbeständigkeit der Folie durch die PET/SiOX-Funktionsschicht kein Problem ist.

### (b) Heißdampfsterilisierte Proben

Die Schweißkurven (s. Figur 2) der Folien der Beispiele 1, 2 und 3 sind ähnlich denen der unsterilisierten Proben, d.h. sie weisen bei ca. 130°C bzw. 135°C eine SNF von >30N/15mm auf; die Folie von Beispiel 4 (zweite Polymerschicht auf LDPE-Basis) zeigt nach dem Heißdampfsterilisieren nicht die gewünschte Schweißnahtfestigkeit. Die Schweißnaht fällt auseinander bzw. "peelt" auf oder delaminiert. Bei Verwendung von Folien gemäß Beispiel 4 als medizinische Beutel würden sich die Beutel beim Sterilisieren im Autoklav oder beim Herausnehmen öffnen.

PE-Materialien wie LDPE oder HDPE sind nicht kompatibel mit Polypropylen, d.h. - im Fall von Mehrschichtfolien mit Mittelschichten auf PP-Basis - die Innenschicht trennt sich leicht von der Mittelschicht. Darüber hinaus liegt die Schmelztemperatur von LDPE-Rohstoffen bei 110 bis 115°C, d.h. der Rohstoff schmilzt während der Heißdampfsterilisation und schwächt die Schweißnaht.

Es zeigt sich, dass die erfindungsgemäße Mehrschichtfolie gemäß den Beispielen 1 und 2, bedingt durch die für die zweite Polymerschicht (Innenschicht) B) verwendete spezifische Kombination eines Composites B1) auf Polyethylen-Basis, eines PP-Terpolymer B2) und eines Polyethylen-Elastomers B3) (als Haftvermittler), den Vorteil hat, dass die Innenschicht B) sich nicht leicht von der Mittelschicht C) ablösen lässt, ferner das verwendete Composite B1) während der Heißdampfsterilisation nicht aufschmilzt und somit die Schweißnahtfestigkeit ausreichend hoch bleibt.

Gegenüber den Mehrschichtfolien des Standes der Technik gemäß Beispiel 3 (Innenschicht B aus schlagzähmodifiziertem Propylen-Terpolymer) weisen die Mehrschichtfolien der erfindungsgemäßen Beispiele 1 und 2 auch ohne den Zusatz eines Schlagzähmodifikators eine sehr gute Schlagzähigkeit - bedingt durch das für die Innenschicht B) verwendete Composite B1) auf Polyethylen-Basis - auf, haben eine vergleichbare Schweißnahtfestigkeit und Eignung für die Heißdampfsterilisation. Ferner bieten erfindungsgemäße Mehrschichtfolien gegenüber solchen gemäß Beispiel 3 den Vorteil, dass Wirkstoffe weniger stark an der PE-haltigen Innenschicht haften bleiben, d.h. der sogenannte "Recovery-Wert" ist höher.

## Patentansprüche

1. Hitzesterilisierbare Mehrschichtfolie, umfassend
a) eine erste Polymerschicht (A) enthaltend mindestens ein mit mindestens einem Impact Modifier modifiziertes Propylen-Homopolymer;
b) eine zweite Polymerschicht (B) enthaltend:
B1) 60 bis 85 Gew.-% - bezogen auf (B) - einer homogenen Zusammensetzung (B1) bestehend aus:
B11) 65 bis 85 Gew.-% - bezogen auf (B1) - Ethylen-Homopolymer, und
B12) 15 bis 35 Gew.-%, bevorzugt 20 bis 30 Gew.-%, - bezogen auf (B1) - mindestens eines Ethylen-Copolymers, welches als Comonomer mindestens ein alpha-Olefin mit 4 bis 12 Kohlenstoffatomen enthält, wobei (B1) eine Schmelztemperatur von > 125°C, bestimmt wie in der Beschreibung angegeben, und eine Dichte, bestimmt gemäß DIN EN ISO 1183-1 : 2019-09 - Verfahren B, von 945 bis 960 kg/m³ aufweist;
B2) 11 bis 30 Gew.-% - bezogen auf (B) - mindestens eines Propylen-Terpolymers;
B3) 4 bis 15 Gew.-% - bezogen auf (B) - mindestens eines Polyethylen-Elastomers, welches ein Copolymer von Ethylen mit mindestens einem alpha-Olefin mit 4 bis 12 Kohlenstoffatomen ist;
c) eine mittlere Polymerschicht (C), die sich zwischen der ersten Polymerschicht (A) und der zweiten Polymerschicht (B) befindet, enthaltend:
C1) 61 bis 80 Gew.-% - bezogen auf (C) - mindestens eines Propylen-Terpolymers;
C2) 15 bis 25 Gew.-% - bezogen auf (C) - mindestens eines Styrol-Blockcopolymer-Elastomers;
C3) 5 bis 19 Gew.-% - bezogen auf (C) - mindestens eines Polyethylen-Elastomers, welches ein Copolymer von Ethylen mit mindestens einem alpha-Olefin enthaltend 4 bis 12 Kohlenstoffatome ist.

2. Hitzesterilisierbare Mehrschichtfolie gemäß Anspruch 1, **dadurch gekennzeichnet, dass** die homogene Zusammensetzung B1) besteht aus:
B11) 70 bis 80 Gew.-%, und
B12) 20 bis 30 Gew.-%.

3. Hitzesterilisierbare Mehrschichtfolie gemäß Anspruch 1 oder 2, **dadurch gekennzeichnet, dass** das Ethylen-Copolymer B12) durch Copolymerisation von Ethylen und mindestens einem alpha-Olefin in Gegenwart eines Metallocen-Katalysators hergestellt wird.

4. Hitzesterilisierbare Mehrschichtfolie gemäß einem der Ansprüche 1 bis 3, worin als Ethylen-Homopolymer B11) ein High-Density-Polyethylen (HDPE) eingesetzt wird, vorzugsweise ein HDPE mit einer Dichte von 950 bis 970 kg/m³.

5. Hitzesterilisierbare Mehrschichtfolie gemäß einem der Ansprüche 1 bis 4, **dadurch gekennzeichnet, dass** das Ethylen-Copolymer B12) 25 bis 40 Gew.-%, bevorzugt 30 bis 37 Gew.-%, mindestens eines alpha-Olefin-Comonomer enthält.

6. Hitzesterilisierbare Mehrschichtfolie gemäß einem der Ansprüche 1 bis 5, **dadurch gekennzeichnet, dass** das Ethylen-Copolymer B12) als Comonomer mindestens ein alpha-Olefin ausgewählt aus 1-Buten, 1-Penten, 1-Hexen und 4-Methyl-1-penten, vorzugsweise 1-Buten, enthält.

7. Hitzesterilisierbare Mehrschichtfolie gemäß einem der Ansprüche 1 bis 6, worin die Komponente B2) und/oder C1) ein Terpolymer von Propylen, Ethylen und/oder C₄ bis C₁₆-α-Olefinen ist, bevorzugt ein Terpolymer von Propylen, Ethylen und Butylen.

8. Hitzesterilisierbare Mehrschichtfolie gemäß einem der Ansprüche 1 bis 7, worin die Komponente B3) ein Copolymer von Ethylen mit einem alpha-Olefin mit 7 bis 12 Kohlenstoffatomen ist.

9. Hitzesterilisierbare Mehrschichtfolie gemäß einem der Ansprüche 1 bis 8, worin die zweite Polymerschicht (B) enthält:
B1) 65 bis 80 Gew.-%, bevorzugt 72 bis 78 Gew.-%,
B2) 15 bis 25 Gew.-%, bevorzugt 17 bis 22 Gew.-%;
B3) 4 bis 12 Gew.-%, bevorzugt 5 bis 10 Gew.-%.

10. Hitzesterilisierbare Mehrschichtfolie gemäß einem der Ansprüche 1 bis 9, worin die mittlere Polymerschicht (C) enthält:
C1) 65 bis 75 Gew.-%;
C2) 17 bis 22 Gew.-%;
C3) 8 bis 17 Gew.-%.

11. Hitzesterilisierbare Mehrschichtfolie gemäß einem der Ansprüche 1 bis 10, worin das Styrol-Blockcopolymer (SBC) Elastomer C2) ausgewählt ist aus der Gruppe bestehend aus: Styrol-Ethylen-Butylen-Styrol-Blockcopolymer (SEBS), Styrol-Ethylen-Propylen-Styrol-Blockcopolymer (SEPS), Styrol-Ethylen-Ethylen-Propylen-Styrol-Blockcopolymer (SEEPS), Styrol-Isopren-Styrol-Blockcopolymer (SIS) und Styrol-Butadien-Styrol-Blockcopolymer (SBS), bevorzugt SEBS und SEPS, insbesondere SEBS.

12. Hitzesterilisierbare Mehrschichtfolie gemäß einem der Ansprüche 1 bis 11, worin das Polyethylen-Elastomer C3) ein Ethylen-Butylen-Copolymer und/oder ein Ethylen-Octen-Copolymer, insbesondere ein Ethylen-Octen-Copolymer ist.

13. Hitzesterilisierbare Mehrschichtfolie gemäß einem der Ansprüche 1 bis 12, **dadurch gekennzeichnet, dass** die Mehrschichtfolie aus den Polymerschichten (A), (B) und (C) besteht, und die gesamte Folienstärke der Mehrschichtfolie 50 bis 500 µm, bevorzugt 100 bis 400 µm, beträgt, und - jeweils bezogen auf die gesamte Folienstärke der Mehrschichtfolie -
die Schichtdicke der ersten Polymerschicht (A) 5 bis 15 Gew.-%, bevorzugt 7 bis 13 Gew.-%;
die Schichtdicke der zweiten Polymerschicht (B) 5 bis 15 Gew.-%, bevorzugt 7 bis 13 Gew.-%; und
die Schichtdicke der mittleren Polymerschicht (C) 70 bis 90 Gew.-%, bevorzugt 74 bis 86 Gew.-%; beträgt.

14. Verfahren zur Herstellung einer hitzesterilisierbaren Mehrschichtfolie gemäß einem der Ansprüche 1 bis 13, wobei die erste Polymerschicht (A), die mittlere Polymerschicht (C), und die zweite Polymerschicht (B) coextrudiert werden.

15. Hitzesterilisierbare Mehrschichtfolie gemäß einem der Ansprüche 1 bis 12, welche eine zusätzliche Funktionsschicht (D) umfasst, die sich benachbart zu der ersten Polymerschicht (A) auf der äußeren Seite von (A) befindet, und wobei Funktionsschicht (D) mindestens ein Material enthält, ausgewählt aus der Gruppe bestehend aus: Ethylen-Vinylalkohol-Copolymeren, Polyvinylalkoholen, Polyamiden, flüssigkristalllinen Polymeren (LCP), aromatischen Polyestern, bevorzugt Terephthalsäure-Polyestern, besonders bevorzugt Polyethylentherephthalaten (PET), Siliziumoxid (SiOₓ), Aluminiumoxid (AlOₓ) und Polymeren auf Acyrylatbasis.

16. Hitzesterilisierbare Mehrschichtfolie gemäß Anspruch 15, worin Funktionsschicht (D) aus PET/SiOₓbesteht.

17. Verfahren zur Herstellung eines kaschierten Mehrschichtfolienschlauches aus einer hitzesterilisierbaren Mehrschichtfolie gemäß einem der Ansprüche 15 oder 16 umfassend die folgenden Schritte:
(a`) Herstellen eines Folienschlauches aus einer gemäß Anspruch 14 hergestellten Mehrschichtfolie, wobei der Innenraum des Folienschlauches mit - vorzugsweise steril gefilterter - Luft geflutet wird;
(b') gegebenenfalls Abkühlen des in Verfahrensschritt (a') hergestellten Folienschlauches;
(c') Beschichten des gegebenenfalls abgekühlten Folienschlauches mit einer Haftkleberadhäsivschicht auf mindestens einer Seite (erste Polymerschicht (A)) des Folienschlauches;
(d') gegebenenfalls Trocknen des mit der Haftkleberadhäsivschicht versehenen Folienschlauches;
(e') Kaschieren der mindestens einen mit der Haftkleberadhäsivschicht beschichteten Seite (erste Polymerschicht (A)) des Folienschlauches mit einer Funktionsschicht (D), insbesondere einer SiOx/PET-Funktionsschicht;
(f) gegebenenfalls Trocknen bzw. Aushärten des kaschierten Folienschlauches.

18. Verwendung einer hitzesterilisierbaren Mehrschichtfolie gemäß einem der Ansprüche 1 bis 13, 15 oder 16 zur Herstellung einer medizinischen Verpackung, vorzugsweise eines medizinischen Beutels.

19. Verfahren zur Herstellung einer medizinischen Verpackung, bevorzugt eines Beutels, aus einer hitzesterilisierbaren Mehrschichtfolie gemäß einem der Ansprüche 1 bis 13, 15 oder 16 umfassend die Schritte:
a) Bereitstellen mindestens einer gemäß Anspruch 14 oder 17 hergestellten Mehrschichtfolie;
b) gegebenenfalls Bereitstellen eines oder mehrerer Portelemente und/oder Schläuche;
c) Formen einer medizinischen Verpackung, bevorzugt eines Beutels, aus der mindestens einen Mehrschichtfolie, so dass die zweite Polymerschicht (B) die innere Fläche der medizinischen Verpackung, bevorzugt des Beutels, und die erste Polymerschicht (A) die äußere Fläche der medizinischen Verpackung, bevorzugt des Beutels, bildet;
d) gegebenenfalls Positionieren der Portelemente und/oder Schläuche zwischen den inneren Flächen an den Konturen der medizinischen Verpackung, bevorzugt des Beutels;
e) In-Kontakt-Bringen der inneren Flächen an den Konturen der medizinischen Verpackung, bevorzugt des Beutels, miteinander und mit gegebenenfalls dazwischen positionierten Portelementen und/oder Schläuchen;
f) Verschweißen der inneren Flächen an den Konturen der medizinischen Verpackung, bevorzugt des Beutels, miteinander und mit gegebenenfalls dazwischen positionierten Portelementen und/oder Schläuchen.

## Claims

1. Heat-sterilizable multilayer film comprising
a) a first polymer layer (A) containing at least one propylene homopolymer modified with at least one impact modifier;
b) a second polymer layer (B) containing:
B1) 60% to 85% by weight - based on (B) - of a homogeneous composition (B1) consisting of:
B11) 65% to 85% by weight - based on (B1) - of ethylene homopolymer, and
B12) 15% to 35% by weight, preferably 20% to 30% by weight - based on (B1) - of at least one ethylene copolymer which contains as comonomer at least one alpha-olefin having 4 to 12 carbon atoms,
where (B1) has a melting temperature of > 125°C, determined as specified in the description, and a density, determined according to DIN EN ISO 1183-1 : 2019-09 - Method B, of 945 to 960 kg/m³;
B2) 11% to 30% by weight - based on (B) - of at least one propylene terpolymer;
B3) 4% to 15% by weight - based on (B) - of at least one polyethylene elastomer which is a copolymer of ethylene with at least one alpha-olefin having 4 to 12 carbon atoms;
c) a central polymer layer (C) situated between the first polymer layer (A) and the second polymer layer (B), containing:
C1) 61% to 80% by weight - based on (C) - of at least one propylene terpolymer;
C2) 15% to 25% by weight - based on (C) - of at least one styrene block copolymer elastomer;
C3) 5% to 19% by weight - based on (C) - of at least one polyethylene elastomer which is a copolymer of ethylene with at least one alpha-olefin containing 4 to 12 carbon atoms.

2. Heat-sterilizable multilayer film according to claim 1, **characterized in that** the homogeneous composition B1) consists of:
B11) 70% to 80% by weight, and
B12) 20% to 30% by weight.

3. Heat-sterilizable multilayer film according to claim 1 or 2, **characterized in that** the ethylene copolymer B12) is prepared by copolymerization of ethylene and at least one alpha-olefin in the presence of a metallocene catalyst.

4. Heat-sterilizable multilayer film according to any of claims 1 to 3, in which the ethylene homopolymer B11) used is a high-density polyethylene (HDPE), preferably an HDPE having a density of 950 to 970 kg/m³.

5. Heat-sterilizable multilayer film according to any of claims 1 to 4, **characterized in that** the ethylene copolymer B12) contains 25% to 40% by weight, preferably 30% to 37% by weight, of at least one alpha-olefin comonomer.

6. Heat-sterilizable multilayer film according to any of claims 1 to 5, **characterized in that** the ethylene copolymer B12) contains as comonomer at least one alpha-olefin selected from 1-butene, 1-pentene, 1-hexene and 4-methyl-1-pentene, preferably 1-butene.

7. Heat-sterilizable multilayer film according to any of claims 1 to 6, in which component B2) and/or C1) is a terpolymer of propylene, ethylene and/or C₄ to C₁₆ α-olefins, preferably a terpolymer of propylene, ethylene and butylene.

8. Heat-sterilizable multilayer film according to any of claims 1 to 7, in which component B3) is a copolymer of ethylene with an alpha-olefin having 7 to 12 carbon atoms.

9. Heat-sterilizable multilayer film according to any of claims 1 to 8, in which the second polymer layer (B) contains:
B1) 65% to 80% by weight, preferably 72% to 78% by weight,
B2) 15% to 25% by weight, preferably 17% to 22% by weight;
B3) 4% to 12% by weight, preferably 5% to 10% by weight.

10. Heat-sterilizable multilayer film according to any of claims 1 to 9, in which the central polymer layer (C) contains:
C1) 65% to 75% by weight;
C2) 17% to 22% by weight;
C3) 8% to 17% by weight.

11. Heat-sterilizable multilayer film according to any of claims 1 to 10, in which the styrene block copolymer (SBC) elastomer C2) is selected from the group consisting of: styrene-ethylene-butylene-styrene block copolymer (SEBS), styrene-ethylene-propylene-styrene block copolymer (SEPS), styrene-ethylene-ethylene-propylene-styrene block copolymer *(*SEEPS*),* styrene-isoprene-styrene block copolymer (SIS) and styrene-butadiene-styrene block copolymer (SBS), preferably SEBS and SEPS, in particular SEBS.

12. Heat-sterilizable multilayer film according to any of claims 1 to 11, in which the polyethylene elastomer C3) is an ethylene-butylene copolymer and/or an ethylene-octene copolymer, in particular an ethylene-octene copolymer.

13. Heat-sterilizable multilayer film according to any of claims 1 to 12, **characterized in that** the multilayer film consists of the polymer layers (A), (B) and (C), and the total film thickness of the multilayer film is 50 to 500 µm, preferably 100 to 400 µm, and - based in each case on the total film thickness of the multilayer film -
the layer thickness of the first polymer layer (A) is 5% to 15% by weight, preferably 7% to 13% by weight;
the layer thickness of the second polymer layer (B) is 5% to 15% by weight, preferably 7% to 13% by weight; and
the layer thickness of the central polymer layer (C) is 70% to 90% by weight, preferably 74% to 86% by weight.

14. Method for producing a heat-sterilizable multilayer film according to any of claims 1 to 13, wherein the first polymer layer (A), the central polymer layer (C) and the second polymer layer (B) are coextruded.

15. Heat-sterilizable multilayer film according to any of claims 1 to 12, which comprises an additional functional layer (D) adjacent to the first polymer layer (A) on the outer side of (A) and wherein functional layer (D) contains at least one material selected from the group consisting of: ethylene-vinyl alcohol copolymers, polyvinyl alcohols, polyamides, liquid crystal polymers (LCP), aromatic polyesters, preferably terephthalic acid polyesters, particularly preferably polyethylene terephthalates (PET), silicon oxide (SiOₓ), aluminum oxide (AlOₓ) and acrylate-based polymers.

16. Heat-sterilizable multilayer film according to claim 15, in which functional layer (D) consists of PET/SiOₓ.

17. Method for producing a laminated multilayer film sleeve made of a heat-sterilizable multilayer film according to any of claims 15 or 16, comprising the following steps:
(a`) producing a film sleeve made of a multilayer film produced according to claim 14, wherein the interior of the film sleeve is flooded with - preferably sterile-filtered - air;
(b') optionally cooling the film sleeve produced in method step (a');
(c') coating the optionally cooled film sleeve with a pressure-sensitive adhesive layer on at least one side (first polymer layer (A)) of the film sleeve;
(d') optionally drying the film sleeve provided with the pressure-sensitive adhesive layer;
(e') laminating the at least one side (first polymer layer (A)) of the film sleeve coated with the pressure-sensitive adhesive layer with a functional layer (D), in particular a SiOx/PET functional layer;
(f) optionally drying or curing the laminated film sleeve.

18. Use of a heat-sterilizable multilayer film according to any of claims 1 to 13, 15 or 16 for production of a medical package, preferably a medical bag.

19. Method for producing a medical package, preferably a bag, made of a heat-sterilizable multilayer film according to any of claims 1 to 13, 15 or 16, comprising the steps of:
a) providing at least one multilayer film produced according to claim 14 or 17;
b) optionally providing one or more port elements and/or flexible tubes;
c) shaping a medical package, preferably a bag, from the at least one multilayer film, such that the second polymer layer (B) forms the inner face of the medical package, preferably the bag, and the first polymer layer (A) forms the outer face of the medical package, preferably the bag;
d) optionally positioning the port elements and/or flexible tubes between the inner faces at the contours of the medical package, preferably the bag;
e) contacting the inner faces at the contours of the medical package, preferably the bag, with one another and with port elements and/or flexible tubes optionally positioned in between;
f) heat-sealing the inner faces at the contours of the medical package, preferably the bag, with one another and with port elements and/or flexible tubes optionally positioned in between.

## Revendications

1. Feuille multicouche stérilisable à la chaleur, comprenant
a) une première couche polymère (A) contenant au moins un homopolymère de propylène modifié par au moins un modificateur d'impact ;
b) une deuxième couche polymère (B) contenant :
B1) 60 à 85% en poids - par rapport à (B) - d'une composition homogène (B1) constituée par :
B11) 65 à 85% en poids - par rapport à (B1) - d'homopolymère d'éthylène et
B12) 15 à 35% en poids, de préférence 20 à 30% en poids - par rapport à (B1) - d'au moins un copolymère d'éthylène qui contient comme comonomère au moins une alpha-oléfine comprenant 4 à 12 atomes de carbone,
(B1) présentant une température de fusion > 125°C, déterminée comme indiqué dans la description, et une masse volumique, déterminée selon la norme DIN EN ISO 1183-1:2019-09 - méthode B, de 945 à 960 kg/m³ ;
B2) 11 à 30% en poids - par rapport à (B) - d'au moins un terpolymère de propylène ;
B3) 4 à 15% en poids - par rapport à (B) - d'au moins un élastomère de polyéthylène, qui est un copolymère d'éthylène avec au moins une alpha-oléfine comprenant 4 à 12 atomes de carbone ;
c) une couche polymère centrale (C), qui se trouve entre la première couche polymère (A) et la deuxième couche polymère (B) contenant :
C1) 61 à 80% en poids - par rapport à (C) - d'au moins un terpolymère de propylène ;
C2) 15 à 25% en poids - par rapport à (C) - d'au moins un élastomère de copolymère séquencé de styrène ;
C3) 5 à 19% en poids - par rapport à (C) - d'au moins un élastomère de polyéthylène, qui est un copolymère d'éthylène avec au moins une alpha-oléfine comprenant 4 à 12 atomes de carbone.

2. Feuille multicouche stérilisable à la chaleur selon la revendication 1, **caractérisée en ce que** la composition homogène B1) est constituée par :
B11) 70 à 80% en poids et
B12) 20 à 30% en poids.

3. Feuille multicouche stérilisable à la chaleur selon la revendication 1 ou 2, **caractérisée en ce que** le copolymère d'éthylène B12) est préparé par copolymérisation d'éthylène et d'au moins une alpha-oléfine en présence d'un catalyseur de type métallocène.

4. Feuille multicouche stérilisable à la chaleur selon l'une des revendications 1 à 3, dans laquelle est utilisé, en tant qu'homopolymère d'éthylène B11), un polyéthylène haute densité (HDPE), de préférence un HDPE présentant une masse volumique de 950 à 970 kg/m³.

5. Feuille multicouche stérilisable à la chaleur selon l'une des revendications 1 à 4, **caractérisée en ce que** le copolymère d'éthylène B12) contient 25 à 40% en poids, de préférence 30 à 37% en poids, d'au moins un comonomère d'alpha-oléfine.

6. Feuille multicouche stérilisable à la chaleur selon l'une des revendications 1 à 5, **caractérisée en ce que** le copolymère d'éthylène B12) contient, comme comonomère, au moins une alpha-oléfine choisie parmi le 1-butène, le 1-pentène, le 1-hexène et le 4-méthyl-1-pentène, de préférence le 1-butène.

7. Feuille multicouche stérilisable à la chaleur selon l'une des revendications 1 à 6, dans laquelle le composant B2) et/ou C1) est un terpolymère de propylène, d'éthylène et/ou de C₄-C₁₆-α-oléfines, de préférence un terpolymère de propylène, d'éthylène et de butylène.

8. Feuille multicouche stérilisable à la chaleur selon l'une des revendications 1 à 7, dans laquelle le composant B3) est un copolymère d'éthylène avec une alpha-oléfine comprenant 7 à 12 atomes de carbone.

9. Feuille multicouche stérilisable à la chaleur selon l'une des revendications 1 à 8, dans laquelle la deuxième couche polymère (B) contient :
B1) 65 à 80% en poids, de préférence 72 à 78% en poids,
B2) 15 à 25% en poids, de préférence 17 à 22% en poids ;
B3) 4 à 12% en poids, de préférence 5 à 10% en poids.

10. Feuille multicouche stérilisable à la chaleur selon l'une des revendications 1 à 9, dans laquelle la couche polymère centrale (C) contient :
C1) 65 à 75% en poids ;
C2) 17 à 22% en poids ;
C3) 8 à 17% en poids.

11. Feuille multicouche stérilisable à la chaleur selon l'une des revendications 1 à 10, dans laquelle l'élastomère de copolymère séquencé de styrène (SBC) C2) est choisi dans le groupe constitué par : copolymère séquencé de styrène-éthylène-butylène-styrène (SEBS), copolymère séquencé de styrène-éthylène-propylène-styrène (SEPS), copolymère séquencé de styrène-éthylène-éthylène-propylène-styrène (SEEPS), copolymère séquencé de styrène-isoprène-styrène (SIS) et copolymère séquencé de styrène-butadiène-styrène (SBS), de préférence SEBS et SEPS, en particulier SEBS.

12. Feuille multicouche stérilisable à la chaleur selon l'une des revendications 1 à 11, dans laquelle l'élastomère de polyéthylène C3) est un copolymère d'éthylène-butylène et/ou un copolymère d'éthylène-octène, en particulier un copolymère d'éthylène-octène.

13. Feuille multicouche stérilisable à la chaleur selon l'une des revendications 1 à 12, **caractérisée en ce que** la feuille multicouche est constituée par les couches polymères (A), (B) et (C) et l'épaisseur totale de feuille de la feuille multicouche est de 50 à 500 um, de préférence de 100 à 400 µm et - à chaque fois par rapport à l'épaisseur totale de feuille -
l'épaisseur de couche de la première couche polymère (A) représente 5 à 15% en poids, de préférence 7 à 13% en poids ;
l'épaisseur de couche de la deuxième couche polymère (B) représente 5 à 15% en poids, de préférence 7 à 13% en poids ; et
l'épaisseur de couche de la couche polymère centrale (C) représente 70 à 90% en poids, de préférence de 74 à 86% en poids.

14. Procédé de préparation d'une feuille multicouche stérilisable à la chaleur selon l'une des revendications 1 à 13, la première couche polymère (A), la couche polymère centrale (C) et la deuxième couche polymère (B) étant coextrudées.

15. Feuille multicouche stérilisable à la chaleur selon l'une des revendications 1 à 12, qui comprend en une couche fonctionnelle (D) supplémentaire qui est adjacente à la première couche polymère (A) sur la face externe de (A) et la couche fonctionnelle (D) contenant au moins un matériau choisi dans le groupe constitué par : les copolymères d'éthylène-alcool vinylique, les poly(alcools vinyliques), les polyamides, les polymères à cristaux liquides (LCP), les polyesters aromatiques, de préférence les polyesters de l'acide téréphtalique, de manière particulièrement préférée les poly(téréphtalates d'éthylène) (PET), l'oxyde de silicium (SiOₓ), l'oxyde d'aluminium (AlOₓ) et les polymères à base d'acrylate.

16. Feuille multicouche stérilisable à la chaleur selon la revendication 15, dans laquelle la couche fonctionnelle (D) est constituée de PET/SiOₓ.

17. Procédé de fabrication d'un flexible en feuille multicouche contrecollé constitué par une feuille multicouche stérilisable à la chaleur selon l'une des revendications 15 ou 16, comprenant les étapes suivantes :
(a') fabrication d'un flexible en feuille à partir d'une feuille multicouche préparée selon la revendication 14, l'espace interne du flexible multicouche étant rempli d'air - de préférence filtré de manière à être stérile ;
(b') le cas échéant refroidissement du flexible en feuille fabriqué dans l'étape de procédé (a') ;
(c') revêtement du flexible en feuille le cas échéant refroidi par une couche autoadhésive sur au moins une face (première couche polymère (A)) du flexible en feuille ;
(d') le cas échéant séchage du flexible en feuille pourvu de la couche autoadhésive ;
(e') contrecollage de ladite au moins une face revêtue par la couche autoadhésive (première couche polymère (A)) du flexible en feuille par une couche fonctionnelle (D), en particulier une couche fonctionnelle de SiOx/PET ;
(f) le cas échéant séchage ou, selon le cas, durcissement du flexible en feuille contrecollé.

18. Utilisation d'une feuille multicouche stérilisable à la chaleur selon l'une des revendications 1 à 13, 15 ou 16 pour la fabrication d'un emballage médical, de préférence d'une poche médicale.

19. Procédé de fabrication d'un emballage médical, de préférence d'une poche, à partir d'une feuille multicouche stérilisable à la chaleur selon l'une des revendications 1 à 13, 15 ou 16 comprenant les étapes :
a) mise à disposition d'au moins une feuille multicouche préparée selon la revendication 14 ou 17 ;
b) le cas échéant mise à disposition d'un ou de plusieurs éléments de portage et/ou flexibles ;
c) formation d'un emballage médical, de préférence d'une poche, à partir de ladite au moins une feuille multicouche, de telle sorte que la deuxième couche polymère (B) forme la surface interne de l'emballage médical, de préférence de la poche, et la première couche polymère (A) forme la surface externe de l'emballage médical, de préférence de la poche ;
d) le cas échéant positionnement des éléments de portage et/ou des flexibles entre les surfaces internes au niveau des contours de l'emballage médical, de préférence de la poche ;
e) mise en contact des surfaces internes au niveau des contours de l'emballage médical, de préférence de la poche, les unes avec les autres et avec les éléments de portage et/ou les flexibles le cas échéant positionnés entre elles ;
f) soudage des surfaces internes au niveau des contours de l'emballage médical, de préférence de la poche, les unes avec les autres et avec les éléments de portage et/ou les flexibles le cas échéant positionnés entre elles.
